(19) Europäisches Patentamt
European Patent Office
Office européen des brevets

(11) EP 4 403 189 A1

(12) **EUROPEAN PATENT APPLICATION**
published in accordance with Art. 153(4) EPC

(43) Date of publication:
**24.07.2024 Bulletin 2024/30**

(21) Application number: **22869309.9**

(22) Date of filing: **15.09.2022**

(51) International Patent Classification (IPC):
***A61K 47/68*** (2017.01) ***A61K 38/07*** (2006.01)
***A61K 39/395*** (2006.01) ***C07K 14/71*** (2006.01)
***A61P 35/00*** (2006.01)

(52) Cooperative Patent Classification (CPC):
**A61K 38/07; A61K 39/395; A61K 47/68; A61P 35/00; C07K 14/71**

(86) International application number:
**PCT/CN2022/118964**

(87) International publication number:
**WO 2023/040941 (23.03.2023 Gazette 2023/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.09.2021 CN 202111088584**

(71) Applicant: **Shanghai Miracogen Inc.**
**Shanghai 201203 (CN)**

(72) Inventors:
- **LI, Hu**
  **Shanghai 201203 (CN)**
- **HU, Chaohong**
  **Shanghai 201203 (CN)**
- **LIU, Wenchao**
  **Shanghai 201203 (CN)**

(74) Representative: **Cabinet Nony**
**11 rue Saint-Georges**
**75009 Paris (FR)**

Remarks:
The complete document including Reference Table(s) and the Sequence Listing(s) can be downloaded from the EPO website

# (54) USE OF ANTIBODY-DRUG CONJUGATE, AND COMBINED DRUG AND USE THEREOF

(57) The present application relates to the use of an antibody-drug conjugate, and a combined drug and the use thereof. Specifically provided are the use of an antibody-drug conjugate, a combined drug and the use thereof. The antibody-drug conjugate shows a significant pharmacodynamic effect of inhibiting tumor cell growth in various NSCLC cell lines with EGFR mutation expression and various human NSCLC PDX tumor models of AZD9291 drug resistance with EGFR mutation expression. In addition, the combined administration of the antibody-drug conjugate and an anti-PD-1 antibody or an anti-PD-L1 antibody shows a significant synergistic pharmacodynamic effect of inhibiting tumor cell growth in the human NSCLC PDX tumor model of AZD9291 drug resistance with EGFR mutation expression.

EP 4 403 189 A1

## Description

### Field of the Invention

The present invention relates to the field of biomedicine, in particular to use of an antibody-drug conjugate, a combination drug and use thereof.

### Background of the Invention

The epidermal growth factor receptor (EGFR) is a transmembrane receptor tyrosine kinase (RTK) in the EGF receptor family and consists of four closely related RTKs: ErbB 1/EGFR, ErbB2/HER2/neu, ErbB3/HER3, and ErbB4/HER4. The EGFR is overexpressed in a variety of human tumors, including colorectal cancer, head and neck cancer, breast cancer, lung cancer, prostate cancer, kidney cancer, pancreatic cancer, ovarian cancer, brain cancer and bladder cancer. In these tumors, the EGFR is involved in signaling cascades that regulate cancer cell growth, signal transduction, differentiation, adhesion, migration, and survival.

Due to its multidimensional role in cancer, the EGFR has become an attractive therapeutic target. Many EGFR-targeted small-molecule tyrosine kinase inhibitors (TKIs, such as gefitinib, erlotinib, and osimertinib) and monoclonal antibodies (such as cetuximab and panitumumab) have been approved as drugs for monotherapy or in combination with chemotherapy. Despite the success of these existing targeted therapies, patients often develop resistance to small-molecule EGFR inhibitors due to mutations within the EGFR kinase domain. In addition, so far, marketed monoclonal antibodies such as cetuximab are only approved for the treatment of KRAS wild-type metastatic colorectal cancer (RAS wt mCRC) and/or squamous cell carcinoma of the head and neck (SCCHN), and panitumumab is approved to treat metastatic colon cancer.

The human EGFR gene is located on chromosomes 7p12-14 and consists of 24 exons. Exons 18-24 encode the TK functional region, wherein exons 18-20 encode N-lobe, and exons 21-24 encode C-lobe. Mutations related to TKI drug sensitivity are concentrated in the exons 18-21. About 49% of the mutations are deletion mutations (e.g., DelE746-A750) in the region of codons 746 to 752 of exon 19, and more than 80% of them have leucine (L)-arginine (R)-glutamic acid (E)-alanine (A) motif missing. About 45% of the mutations are point mutations L858R in exon 21 (mutation of leucine at position 858 to arginine), and other less frequent mutations include G719S in exon 18 and L861Q in exon 21.

Studies have shown that about 50-60% of patients with acquired TKI-resistant NSCLC have T790M mutation and do not respond to gefitinib or erlotinib. Even treated with osimertinib, a third-generation TKI that specifically targets the T790M mutation, NSCLC patients still experience disease progression after about 10 months of treatment.

For non-small cell lung cancer (NSCLC), although the invention of TKIs has greatly transformed the treatment landscape and benefited these patients, the speed at which mutations occur in the EGFR kinase domain far outpaces the development of new TKIs to overcome the resistance caused by these mutations. Drug resistance caused by EGFR mutations is one of the main challenges of current EGFR-targeted TKIs. Therefore, currently available EGFR-targeted treatments cannot fully meet the needs of patients, and there is an obvious unmet medical need for treatments with new mechanisms.

Immune checkpoint inhibitors targeting the PD-1/PD-L1 pathway have shown significant and durable clinical responses in patients with several cancers, including NSCLC. Although PD-1/PD-L1 checkpoint blockade can lead to dramatic responses, this therapy is only effective in a subset of patients, and many patients respond only partially to the treatment. Clinical trials have shown that the objective response rate to anti-PD-1 antibodies in squamous NSCLC patients is approximately 33%, and the objective response rate to anti-PD-1 antibodies in non-squamous NSCLC patients is 12%. In addition, the application of PD-1/PD-L1 inhibitors in NSCLC carrying EGFR-activating mutations is still unclear at present. It is unclear whether EGFR-mutated NSCLC can benefit from PD-1/PD-L1 inhibitors.

In view of the above-mentioned drug resistance problems existed in existing targeted EGFR-TKI drugs and the limitations of PD-1/PD-L1 checkpoint blockade therapy, it is still urgent to provide a treatment method with better efficacy, lower toxic and side effects, and less drug resistance.

### Summary of the Invention

It is found that the antibody-drug conjugate (such as MYK-3) of the present invention has shown significant pharmacodynamic effects in inhibiting the growth of tumor cells in various NSCLC cell lines expressing EGFR mutations, as well as various AZD9291 (Osimertinib)-resistant human NSCLC PDX tumor models expressing EGFR mutations.

In addition, it is also found that the combined use of the antibody-drug conjugate of the present invention (such as MYK-3) and an anti-PD-1 antibody (such as AK103) shows significant synergistic pharmacodynamic effects in inhibiting the growth of tumor cells in AZD9291 (Osimertinib)-resistant human NSCLC PDX tumor models expressing EGFR

mutations.

In view of this, in the first aspect of the present invention, provided is use of an antibody-drug conjugate, a pharmaceutically acceptable salt or solvate of the antibody-drug conjugate or a solvate of the salt, which is one or more selected from the group consisting of:

1) in the preparation of a non-small cell lung cancer inhibitor; and
2) in the preparation of a medicament for treating and/or preventing non-small cell lung cancer;

wherein, the non-small cell lung cancer is one or more selected from the group consisting of:

1) EGFR-TKI-resistant non-small cell lung cancer; and
2) EGFR mutated non-small cell lung cancer, and optionally, the EGFR mutated non-small cell lung cancer also has one or more selected from the group consisting of: CDKN2A/2B mutation, STK11 mutation, c-Met amplification, and HER3 amplification;

wherein the antibody-drug conjugate has a structure of formula I,

$$Ab\text{-}(L\text{-}D)_p \qquad \text{formula I}$$

wherein:

Ab represents an anti-EGFR antibody, wherein the anti-EGFR antibody comprises a heavy chain and a light chain, wherein CDR1, CDR2, and CDR3 in a heavy chain variable region respectively comprise sequences as shown in SEQ ID NOs: 5-7 or mutants thereof, and CDR1, CDR2, and CDR3 in a light chain variable region respectively comprise sequences as shown in SEQ ID NOs: 12-14 or mutants thereof;
L represents a linker;
D represents a cytotoxic agent;
p represents 1-9 (such as 1, 2, 3, 4, 5, 6, 7, 8 or 9, or such as 1-7), such as 2-6, 3-5, specifically, such as 3.9, 4.0 or 4.1.

In the second aspect of the present invention, provided is an antibody-drug conjugate, a pharmaceutically acceptable salt or solvate of the antibody-drug conjugate or a solvate of the salt, for use in one or more of the following:

1) inhibiting the growth of a non-small cell lung cancer tumor; and
2) treating and/or preventing non-small cell lung cancer,

wherein, the non-small cell lung cancer is one or more selected from the group consisting of:

1) EGFR-TKI-resistant non-small cell lung cancer; and
2) EGFR mutated non-small cell lung cancer, and optionally, the EGFR mutated non-small cell lung cancer also has one or more selected from the group consisting of: CDKN2A/2B mutation, STK11 mutation, c-Met amplification, and HER3 amplification;

wherein the antibody-drug conjugate has a structure of formula I,

$$Ab\text{-}(L\text{-}D)_p \qquad \text{formula I}$$

wherein:

Ab represents an anti-EGFR antibody, wherein the anti-EGFR antibody comprises a heavy chain and a light chain, wherein CDR1, CDR2, and CDR3 in a heavy chain variable region respectively comprise sequences as shown in SEQ ID NOs: 5-7 or mutants thereof, and CDR1, CDR2, and CDR3 in a light chain variable region respectively comprise sequences as shown in SEQ ID NOs: 12-14 or mutants thereof;
L represents a linker;
D represents a cytotoxic agent;
p represents 1-9 (such as 1, 2, 3, 4, 5, 6, 7, 8 or 9, or such as 1-7), such as 2-6, 3-5, specifically, such as 3.9, 4.0 or 4.1.

In the third aspect of the present invention, provided is a method for inhibiting the growth of a non-small cell lung cancer tumor, and/or a method for treating and/or preventing non-small cell lung cancer, comprising: administering

to a subject in need thereof an effective amount of an antibody-drug conjugate, a pharmaceutically acceptable salt or solvate of the antibody-drug conjugate or a solvate of the salt,
wherein, the non-small cell lung cancer is one or more selected from the group consisting of:

1) EGFR-TKI-resistant non-small cell lung cancer; and
2) EGFR mutated non-small cell lung cancer, and optionally, the EGFR mutated non-small cell lung cancer also has one or more selected from the group consisting of: CDKN2A/2B mutation, STK11 mutation, c-Met amplification, and HER3 amplification;

wherein the antibody-drug conjugate has a structure of formula I,

$$Ab\text{-}(L\text{-}D)_p \qquad \text{formula I}$$

wherein:

Ab represents an anti-EGFR antibody, wherein the anti-EGFR antibody comprises a heavy chain and a light chain, wherein CDR1, CDR2, and CDR3 in a heavy chain variable region respectively comprise sequences as shown in SEQ ID NOs: 5-7 or mutants thereof, and CDR1, CDR2, and CDR3 in a light chain variable region respectively comprise sequences as shown in SEQ ID NOs: 12-14 or mutants thereof;
L represents a linker;
D represents a cytotoxic agent;
p represents 1-9 (such as 1, 2, 3, 4, 5, 6, 7, 8 or 9, or such as 1-7), such as 2-6, 3-5, specifically, such as 3.9, 4.0 or 4.1.

In some embodiments, the dose of the antibody-drug conjugate, the pharmaceutically acceptable salt or solvate of the antibody-drug conjugate or the solvate of the salt is 0.1-10.0 mg/kg (such as 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2.0 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.2 mg/kg, 3.4 mg/kg, 3.6 mg/kg, 3.8 mg/kg, 4.0 mg/kg, 4.2 mg/kg, 4.4 mg/kg, 4.6 mg/kg, 4.8 mg/kg, 5.0 mg/kg, 6.2 mg/kg, 6.4 mg/kg, 6.6 mg/kg, 6.8 mg/kg, 7.0 mg/kg, 7.2 mg/kg, 7.4 mg/kg, 7.6 mg/kg, 7.8 mg/kg, 8.0 mg/kg, 8.2 mg/kg, 8.4 mg/kg, 8.6 mg/kg, 8.8 mg/kg, 9.0 mg/kg, 9.2 mg/kg, 9.4 mg/kg, 9.6 mg/kg, 9.8 mg/kg or 10.0 mg/kg, or 0.1-0.3 mg/kg, 0.3-0.5 mg/kg, 0.5-0.7 mg/kg, 0.7-0.9 mg/kg, 0.9-1.0 mg/kg, 1.0-1.3 mg/kg, 1.3-1.5 mg/kg, 1.5-1.7 mg/kg, 1.7-1.9 mg/kg, 1.9-2.0 mg/kg, 2.0-2.3 mg/kg, 2.3-2.5 mg/kg, 2.5-2.7 mg/kg, 2.7-2.9 mg/kg, 2.9-3.0 mg/kg, 3.0-3.3 mg/kg, 3.3-3.5 mg/kg, 3.5-3.7 mg/kg, 3.7-3.9 mg/kg, 3.9-4.0 mg/kg, 4.0-4.3 mg/kg, 4.3-4.5 mg/kg, 4.5-4.7 mg/kg, 4.7-4.9 mg/kg, or 4.9-5.0 mg/kg).
In some embodiments, the dose of the antibody-drug conjugate, the pharmaceutically acceptable salt or solvate of the antibody-drug conjugate or the solvate of the salt is 0.5-2.5 mg/kg.
In some embodiments, the dose of the antibody-drug conjugate, the pharmaceutically acceptable salt or solvate of the antibody-drug conjugate or the solvate of the salt is 2.0-2.5 mg/kg.
In addition, it should be noted that the "antibody-drug conjugate" described above refers to a composition comprising ADC molecules with the same or different DAR.
Specifically, the present invention provides a composition comprising a plurality of anti-EGFR ADC molecules. In some cases, each ADC in the composition described herein comprises the same number of one or more cytotoxic agents. In other cases, each ADC in the composition described herein comprises a different number of one or more cytotoxic agents.
In the antibody-drug conjugate described herein, each anti-EGFR antibody can be conjugated with 1, 2, 3, 4, 5, 6, 7, 8, or more cytotoxic agents.
The drug-to-antibody ratio (DAR) mentioned above refers to the number of molecules of cytotoxic agent conjugated to anti-EGFR antibodies. The number of molecules of cytotoxic agent contained in the ADC described herein is generally an integer, when the number (e.g., p in formula I) of molecules of cytotoxic agent contained in the ADC described herein is a fraction, that fraction refers to the average number of cytotoxic agents conjugated to each anti-EGFR antibody in a composition comprising a plurality of ADC molecules.
In some embodiments, the linker is selected from the group consisting of 6-maleimidocaproyl (MC), maleimidopropionyl (MP), N-succinimidyl 4-(2-pyridylthio) valerate (SPP), 4-(N-maleimidomethyl)-cyclohexan-1-formyl (MCC), N-succinimidyl(4-iodo-acetyl)aminobenzoate (SIAB), and 6-maleimidocaproyl-valine-citrulline-p-aminobenzyloxycarbonyl (MC-vc-PAB).
In some embodiments, the linker is 6-maleimidocaproyl-valine-citrulline-p-aminobenzyloxycarbonyl (MC-vc-PAB).
In some embodiments, the cytotoxic agent is selected from the group consisting of toxins such as SN-38,

Gemcitabine, Monomethyl auristatin E (MMAE), Monomethyl auristatin F (MMAF), maytansinoids (such as Maytansine DM1 and Maytansine DM4), calicheamicin, MGBA (such as duocarmycin), doxorubicin, Ricin and diphtheria toxin, 1131, interleukins, tumor necrosis factors, chemokines and nanoparticles.

In some embodiments, the cytotoxic agent is Monomethyl auristatin E (MMAE).

In some embodiments, FR1, FR2, FR3, and FR4 regions in the heavy chain variable region of the anti-EGFR antibody respectively comprise sequences as shown in SEQ ID NOs: 8-11 or mutants thereof.

In some embodiments, FR1, FR2, FR3, and FR4 regions in the light chain variable region of the anti-EGFR antibody respectively comprise sequences as shown in SEQ ID NOs: 15-18 or mutants thereof.

In some embodiments, the heavy chain constant region of the anti-EGFR antibody is selected from human IgG, IgM, IgA, IgD, and IgA constant regions or mutants thereof.

In some embodiments, the IgG is selected from IgG1, IgG2, IgG3 and IgG4.

In some embodiments, the light chain constant region of the anti-EGFR antibody is selected from human lambda and kappa constant regions or mutants thereof.

In some embodiments, the sequence of the heavy chain variable region of the anti-EGFR antibody comprises a sequence as shown in SEQ ID NO: 1, or a sequence having greater than 70%, preferably greater than 75%, 80%, 85%, 90%, 95%, or 99% identity to the sequence as shown in SEQ ID NO: 1.

In some embodiments, the sequence of the heavy chain variable region of the anti-EGFR antibody is shown in SEQ ID NO: 1.

In some embodiments, the sequence of the light chain variable region of the anti-EGFR antibody comprises a sequence as shown in SEQ ID NO: 2, or a sequence having greater than 70%, preferably greater than 75%, 80%, 85%, 90%, 95%, or 99% identity to the sequence as shown in SEQ ID NO: 2.

In some embodiments, the sequence of the light chain variable region of the anti-EGFR antibody is shown in SEQ ID NO: 2.

In some embodiments, the sequence of the heavy chain constant region of the anti-EGFR antibody comprises a sequence as shown in SEQ ID NO: 3, or a sequence having greater than 70%, preferably greater than 75%, 80%, 85%, 90%, 95%, or 99% identity to the sequence as shown in SEQ ID NO: 3.

In some embodiments, the sequence of the heavy chain constant region of the anti-EGFR antibody is shown in SEQ ID NO: 3.

In some embodiments, the sequence of the light chain constant region of the anti-EGFR antibody comprises a sequence as shown in SEQ ID NO: 4, or a sequence having greater than 70%, preferably greater than 75%, 80%, 85%, 90%, 95%, or 99% identity to the sequence as shown in SEQ ID NO: 4.

In some embodiments, the sequence of the light chain constant region of the anti-EGFR antibody is shown in SEQ ID NO: 4.

In some embodiments, the EGFR-TKI-resistant non-small cell lung cancer has one or more selected from the group consisting of: EGFR mutation, CDKN2A/2B mutation, STK11 mutation, c-Met amplification, and HER3 amplification.

In some embodiments, the EGFR mutation is a mutation at one or more sites selected from the group consisting of: exon 18, exon 19, exon 20, and exon 21.

In some embodiments, the EGFR mutation is one or more mutations selected from the group consisting of: DelE746-A750, L858R, T790M, C797S, G719S, L861Q, G719C, G719A, T854A, and D761Y.

In some embodiments, the EGFR mutation is one or more mutations selected from the group consisting of: DelE746-A750, L858R, T790M, and C797S.

In some embodiments, the EGFR mutation is one or more mutations selected from the group consisting of: DelE746-A750, L858R, and T790M.

In some embodiments, the EGFR mutation is DelE746-A750, L858R, T790M, C797S or L858R/T790M double mutation.

In some embodiments, the EGFR mutation is DelE746-A750, L858R, T790M or L858R/T790M double mutation.

In some embodiments, the EGFR-TKI is one or more selected from the group consisting of : osimertinib (AZD9291), gefitinib, erlotinib, icotinib, afatinib, dacotinib, imatinib, lapatinib, nazartinib, rociletinib, naquotinib, vandetanib, neratinib, pelitinib, canertinib, brigatinib, PKC412, Go6976, mavelertinib, olmutinib, WZ4002, TAS2913, cetuximab, panitumumab, avitinib, HS-10296, and TQB3804.

In some embodiments, the EGFR-TKI is osimertinib (AZD9291).

In some embodiments, the non-small cell lung cancer is selected from the group consisting of lung adenocarcinoma (preferably, bronchoalveolar carcinoma), lung squamous cell carcinoma, adenosquamous carcinoma of the lung, and large cell lung carcinoma.

In the fourth aspect of the present invention, provided is a combination drug, comprising:

a first drug, the first drug being an antibody-drug conjugate, a pharmaceutically acceptable salt or solvate of the

antibody-drug conjugate, or a solvate of the salt; and
a second drug, the second drug being a PD-1/PD-L1 inhibitor;
wherein the antibody-drug conjugate has a structure of formula I,

$$Ab\text{-}(L\text{-}D)_p \qquad \text{formula I}$$

wherein:

Ab represents an anti-EGFR antibody, wherein the anti-EGFR antibody comprises a heavy chain and a light chain, wherein CDR1, CDR2, and CDR3 in a heavy chain variable region respectively comprise sequences as shown in SEQ ID NOs: 5-7 or mutants thereof, and CDR1, CDR2, and CDR3 in a light chain variable region respectively comprise sequences as shown in SEQ ID NOs: 12-14 or mutants thereof;
L represents a linker;
D represents a cytotoxic agent;
p represents 1-9 (such as 1, 2, 3, 4, 5, 6, 7, 8 or 9, or such as 1-7), such as 2-6, 3-5, specifically, such as 3.9, 4.0 or 4.1.

In some embodiments, the first drug and the second drug are separated from each other. It should be noted that being separated from each other means that the first drug and the second drug are not used as a mixture, but are used in a separated state. For example, the first drug and the second drug are used for combined administration (such as simultaneous administration, or sequential administration, or cross-administration).
In some embodiments, the linker is selected from the group consisting of 6-maleimidocaproyl (MC), maleimidopropionyl (MP), N-succinimidyl 4-(2-pyridylthio) valerate (SPP), 4-(N-maleimidomethyl)-cyclohexan-1-formyl (MCC), N-succinimidyl(4-iodo-acetyl)aminobenzoate (SIAB), and 6-maleimidocaproyl-valine-citrulline-p-aminobenzyloxycarbonyl (MC-vc-PAB).
In some embodiments, the linker is 6-maleimidocaproyl-valine-citrulline-p-aminobenzyloxycarbonyl (MC-vc-PAB).
In some embodiments, the cytotoxic agent is selected from the group consisting of toxins such as SN-38, Gemcitabine, Monomethyl auristatin E (MMAE), Monomethyl auristatin F (MMAF), maytansinoids (such as Maytansine DM1 and Maytansine DM4), calicheamicin, MGBA (such as duocarmycin), doxorubicin, Ricin and diphtheria toxin, 1131, interleukins, tumor necrosis factors, chemokines and nanoparticles.
In some embodiments, the cytotoxic agent is Monomethyl auristatin E (MMAE).
In some embodiments, FR1, FR2, FR3, and FR4 regions in the heavy chain variable region of the anti-EGFR antibody respectively comprise sequences as shown in SEQ ID NOs: 8-11 or mutants thereof.
In some embodiments, FR1, FR2, FR3, and FR4 regions in the light chain variable region of the anti-EGFR antibody respectively comprise sequences as shown in SEQ ID NOs: 15-18 or mutants thereof.
In some embodiments, the heavy chain constant region of the anti-EGFR antibody is selected from human IgG, IgM, IgA, IgD, and IgA constant regions or mutants thereof.
In some embodiments, the IgG is selected from IgG1, IgG2, IgG3 and IgG4.
In some embodiments, the light chain constant region of the anti-EGFR antibody is selected from human lambda and kappa constant regions or mutants thereof.
In some embodiments, the sequence of the heavy chain variable region of the anti-EGFR antibody comprises a sequence as shown in SEQ ID NO: 1, or a sequence having greater than 70%, preferably greater than 75%, 80%, 85%, 90%, 95%, or 99% identity to the sequence as shown in SEQ ID NO: 1.
In some embodiments, the sequence of the heavy chain variable region of the anti-EGFR antibody is shown in SEQ ID NO: 1.
In some embodiments, the sequence of the light chain variable region of the anti-EGFR antibody comprises a sequence as shown in SEQ ID NO: 2, or a sequence having greater than 70%, preferably greater than 75%, 80%, 85%, 90%, 95%, or 99% identity to the sequence as shown in SEQ ID NO: 2.
In some embodiments, the sequence of the light chain variable region of the anti-EGFR antibody is shown in SEQ ID NO: 2.
In some embodiments, the sequence of the heavy chain constant region of the anti-EGFR antibody comprises a sequence as shown in SEQ ID NO: 3, or a sequence having greater than 70%, preferably greater than 75%, 80%, 85%, 90%, 95%, or 99% identity to the sequence as shown in SEQ ID NO: 3.
In some embodiments, the sequence of the heavy chain constant region of the anti-EGFR antibody is shown in SEQ ID NO: 3.
In some embodiments, the sequence of the light chain constant region of the anti-EGFR antibody comprises a sequence as shown in SEQ ID NO: 4, or a sequence having greater than 70%, preferably greater than 75%, 80%,

85%, 90%, 95%, or 99% identity to the sequence as shown in SEQ ID NO: 4.

In some embodiments, the sequence of the light chain constant region of the anti-EGFR antibody is shown in SEQ ID NO: 4.

In some embodiments, the PD-1/PD-L1 inhibitor is an anti-PD-1 antibody.

In some embodiments, the anti-PD-1 antibody is one or more selected from the group consisting of: AK103 (HX008), Toripalimab (JS-001), Sintilimab (IBI308), Camrelizumab, Tislelizumab (BGB-A317), Opdivo or Nivolumab, Keytruda or Pembrolizumab, CS1003, serplulimab (HLX10), AK104, geptanolimab (GB226), lizumab (e.g., LZM009), BAT-1306, SCT-I10A, F520, SG001, GLS-010, PDR001, REGN2810, and STI-A1110.

In some embodiments, the anti-PD-1 antibody is AK103 (HX008).

In some embodiments, the PD-1/PD-L1 inhibitor is an anti-PD-L1 antibody.

In some embodiments, the anti-PD-L1 antibody is Durvalumab, Atezolizumab or a combination thereof.

In some embodiments, a weight ratio of the first drug to the second drug is 15:1-1:15 (such as 15:1, 14:1, 13:1, 12:1, 11:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:11, 1:12, 1:13, 1:14 or 1:15,or 15:1-14:1, 14:1-12:1, 12:1-10:1, 10:1-8:1, 8:1-6:1, 6:1-4:1, 4:1-2:1, 2:1-1:2, 1:2-1:4, 1:4-1:6, 1:6-1:8, 1:8-1:10, 1:10-1:12, 1:12-1:14 or 1:14-1:15).

In some embodiments, the weight ratio of the first drug to the second drug is 1:1-1:10.

In some embodiments, the weight ratio of the first drug to the second drug is 1:1-1:9, 1:2-1:8, 1:3-1:7 or 1:4-1:6.

In some embodiments, the weight ratio of the first drug to the second drug is 1:5.

In the fifth aspect of the present invention, provided is use of the combination drug described above, which is one or more selected from the group consisting of:

1) in the preparation of a non-small cell lung cancer inhibitor; and
2) in the preparation of a medicament for treating and/or preventing non-small cell lung cancer;

wherein, the non-small cell lung cancer is one or more selected from the group consisting of:

1) EGFR-TKI-resistant non-small cell lung cancer; and
2) EGFR mutated non-small cell lung cancer, and optionally, the EGFR mutated non-small cell lung cancer also has one or more selected from the group consisting of: CDKN2A/2B mutation, STK11 mutation, c-Met amplification, and HER3 amplification.

In the sixth aspect of the present invention, provided is a combination drug described above, for use in one or more of the following:

1) inhibiting the growth of a non-small cell lung cancer tumor; and
2) treating and/or preventing non-small cell lung cancer;

wherein, the non-small cell lung cancer is one or more selected from the group consisting of:

1) EGFR-TKI-resistant non-small cell lung cancer; and
2) EGFR mutated non-small cell lung cancer, and optionally, the EGFR mutated non-small cell lung cancer also has one or more selected from the group consisting of: CDKN2A/2B mutation, STK11 mutation, c-Met amplification, and HER3 amplification.

In some embodiments, the EGFR-TKI-resistant non-small cell lung cancer has one or more selected from the group consisting of: EGFR mutation, CDKN2A/2B mutation, STK11 mutation, c-Met amplification, and HER3 amplification.

In some embodiments, the EGFR mutation is a mutation at one or more sites selected from the group consisting of: exon 18, exon 19, exon 20, and exon 21.

In some embodiments, the EGFR mutation is one or more mutations selected from the group consisting of: DelE746-A750, L858R, T790M, C797S, G719S, L861Q, G719C, G719A, T854A, and D761Y.

In some embodiments, the EGFR mutation is one or more mutations selected from the group consisting of: DelE746-A750, L858R, and T790M.

In some embodiments, the EGFR mutation is DelE746-A750, L858R, T790M or L858R/T790M double mutation.

In some embodiments, the EGFR-TKI is one or more selected from the group consisting of: osimertinib (AZD9291), gefitinib, erlotinib, icotinib, afatinib, dacotinib, imatinib, lapatinib, nazartinib, rociletinib, naquotinib, vandetanib, neratinib, pelitinib, canertinib, brigatinib, PKC412, Go6976, mavelertinib, olmutinib, WZ4002, TAS2913, cetuximab, panitumumab, avitinib, HS-10296, and TQB3804.

In some embodiments, the EGFR-TKI is osimertinib (AZD9291).

In some embodiments, the non-small cell lung cancer is selected from the group consisting of lung adenocarcinoma (preferably, bronchoalveolar carcinoma), lung squamous cell carcinoma, adenosquamous carcinoma of the lung, and large cell lung carcinoma.

In the seventh aspect of the present invention, provided is a method for inhibiting the growth of a non-small cell lung cancer tumor, and/or a method for treating and/or preventing non-small cell lung cancer, comprising: administering to a subject in need thereof an effective amount of a first drug and an effective amount of a second drug, wherein the first drug is the antibody-drug conjugate as described above or a pharmaceutically acceptable salt or solvate of the antibody-drug conjugate or a solvate of the salt, wherein the second drug is a PD-1/PD-L1 inhibitor; wherein the non-small cell lung cancer is one or more selected from the group consisting of:

1) EGFR-TKI-resistant non-small cell lung cancer; and
2) EGFR mutated non-small cell lung cancer, and optionally, the EGFR mutated non-small cell lung cancer also has one or more selected from the group consisting of: CDKN2A/2B mutation, STK11 mutation, c-Met amplification, and HER3 amplification.

In some embodiments, the PD-1/PD-L1 inhibitor is an anti-PD-1 antibody.

In some embodiments, the anti-PD-1 antibody is one or more selected from the group consisting of: AK103 (HX008), Toripalimab (JS-001), Sintilimab (IBI308), Camrelizumab, Tislelizumab (BGB-A317), Opdivo or Nivolumab, Keytruda or Pembrolizumab, CS1003, serplulimab (HLX10), AK104, geptanolimab (GB226), lizumab (e.g., LZM009), BAT-1306, SCT-I10A, F520, SG001, GLS-010, PDR001, REGN2810, and STI-A1110.

In some embodiments, the anti-PD-1 antibody is AK103 (HX008).

In some embodiments, the PD-1/PD-L1 inhibitor is an anti-PD-L1 antibody.

In some embodiments, the anti-PD-L1 antibody is Durvalumab, Atezolizumab or a combination thereof.

In some embodiments, a dose ratio of the first drug to the second drug is 15:1-1:15 (such as 15:1, 14:1, 13:1, 12:1, 11:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:11, 1:12, 1:13, 1:14 or 1:15, or 15:1-14:1, 14:1-12:1, 12:1-10:1, 10:1-8:1, 8:1-6:1, 6:1-4:1, 4:1-2:1, 2:1-1:2, 1:2-1:4, 1:4-1:6, 1:6-1:8, 1:8-1:10, 1:10-1:12, 1:12-1:14 or 1:14-1:15).

In some embodiments, the dose ratio of the first drug to the second drug is 1:1-1:10.

In some embodiments, the dose ratio of the first drug to the second drug is 1:1-1:9, 1:2-1:8, 1:3-1:7 or 1:4-1:6.

In some embodiments, the dose ratio of the first drug to the second drug is 1:5.

It should be noted that those skilled in the art can understand that the dose of the first drug and of the second drug is the total dose for preventing and treating diseases. When the total dose remains unchanged, there can be different administration modes, such as administration once every X days, and administration Y times, etc.

In some embodiments, the dose of the first drug is 1 mg/kg. In some embodiments, the dose of the second drug is 5 mg/kg.

In some embodiments, the first drug and the second drug are cross-administered.

In some embodiments, the administration mode of the first drug is: from day 0, once every 4 days for a total of 3 times.

In some embodiments, the administration mode of the second drug is: from day 0, once every 3 days for a total of 10 times.

In some embodiments, the administration route of the first drug and the second drug is intravenous injection.

In some embodiments, the EGFR-TKI-resistant non-small cell lung cancer has one or more selected from the group consisting of: EGFR mutation, CDKN2A/2B mutation, STK11 mutation, c-Met amplification, and HER3 amplification.

In some embodiments, the EGFR mutation is a mutation at one or more sites selected from the group consisting of: exon 18, exon 19, exon 20, and exon 21.

In some embodiments, the EGFR mutation is one or more mutations selected from the group consisting of: DelE746-A750, L858R, T790M, C797S, G719S, L861Q, G719C, G719A, T854A, and D761Y.

In some embodiments, the EGFR mutation is one or more mutations selected from the group consisting of: DelE746-A750, L858R, and T790M.

In some embodiments, the EGFR mutation is DelE746-A750, L858R, T790M or L858R/T790M double mutation.

In some embodiments, the EGFR-TKI is one or more selected from the group consisting of : osimertinib (AZD9291), gefitinib, erlotinib, icotinib, afatinib, dacotinib, imatinib, lapatinib, nazartinib, rociletinib, naquotinib, vandetanib, neratinib, pelitinib, canertinib, brigatinib, PKC412, Go6976, mavelertinib, olmutinib, WZ4002, TAS2913, cetuximab, panitumumab, avitinib, HS-10296, and TQB3804.

In some embodiments, the EGFR-TKI is osimertinib (AZD9291).

In some embodiments, the non-small cell lung cancer is selected from the group consisting of lung adenocarcinoma (preferably, bronchoalveolar carcinoma), lung squamous cell carcinoma, adenosquamous carcinoma of the lung, and large cell lung carcinoma.

Beneficial Effect:

1. The antibody-drug conjugate (such as MYK-3) of the present invention has shown significant pharmacodynamic effects in inhibiting the growth of tumor cells in various NSCLC cell lines expressing EGFR mutations, as well as various AZD9291-resistant human NSCLC PDX tumor models expressing EGFR mutations.

2. The combined use of the antibody-drug conjugate of the present invention (such as MYK-3) and an anti-PD-1 antibody (such as AK103) or an anti-PD-L1 antibody shows significant synergistic pharmacodynamic effects in inhibiting the growth of tumor cells in AZD9291-resistant human NSCLC PDX tumor models expressing EGFR mutations.

**Brief Description of the Drawings**

Fig. 1 is a HIC-HPLC chromatogram for determining the drug/antibody ratio of an antibody drug conjugate.

Fig. 2 shows representative cell killing curves of MYK-3 and Erbitux® in human lung cancer cell line NCI-H1975.

Fig. 3 shows representative cell killing curves of MYK-3 and Erbitux® in human lung cancer cell line NCI-H1650.

Fig. 4 shows the efficacy of ADC-3 and Erbitux® on human lung cancer NCI-H1975 subcutaneous xenograft tumors in nude mice.

Fig. 5 shows the effect of various test drugs on tumor volume in AZD9291-resistant human lung cancer PDX model LUN#2005-143.

Fig. 6 shows the effect of various test drugs on tumor volume in AZD9291-resistant human lung cancer PDX model LUN#2210-4a.

Fig. 7 shows the effect of various test drugs on tumor volume in AZD9291-resistant human lung cancer PDX model LUN#2210-106.

Fig. 8 shows the effect of various test drugs on tumor volume in AZD9291-resistant human lung cancer PDX model LUN#2355-128.

Fig. 9 shows the effect of various test drugs on tumor volume in AZD9291-resistant human lung cancer PDX model LUN#2441-118.

Fig. 10 shows the effect of various tested drugs on tumor volume in human lung cancer PDX model LUN#2210-106.

**Detailed Description of the Embodiments**

The embodiments of the disclosure will be described in detail below in conjunction with examples. However, those skilled in the art will understand that the following examples are only used to illustrate the invention, not to limit the scope of the invention. Those without specific conditions in the examples are generally implemented under conventional conditions or conditions recommended by the manufacturers. The reagents or instruments used without specifying the manufacturers are all conventional products that can be purchased commercially.

In the invention, all scientific and technical terms used herein have the meanings commonly understood by those skilled in the art unless specified otherwise. In addition, the related terms of protein and nucleic acid chemistry, molecular biology, cell and tissue culture, microbiology, immunology, and laboratory procedures used herein are all terms and routine procedures widely used in the corresponding art. Moreover, definitions and explanations of related terms are provided below to understand the invention better.

Unless otherwise stated, in the invention, any concentration range, percentage range, ratio range or numerical range shall be understood to include any integer value within the stated range and, where appropriate, fractional values within the stated range.

The term "antibody", as used herein, refers to an immunoglobulin molecule usually composed of two pairs of identical polypeptide chains, each pair having a "light" (L) chain and a "heavy" (H) chain. The light chains of the antibody can be divided into two categories: κ and λ. The heavy chains can be divided into five categories: μ, δ, γ, α or ε. According to the difference of heavy chains, antibodies can be divided into five categories: IgM, IgD, IgG, IgA and IgE. Within the light and heavy chains, the variable and constant regions are connected by a "J" region of approximately 12 or more amino acids, and the heavy chain also has a "D" region of approximately 3 or more amino acids. Each heavy chain consists of a heavy chain variable region ($V_H$) and a heavy chain constant region ($C_H$). The heavy chain constant region consists of three domains ($C_H1$, $C_H2$ and $C_H3$). Each light chain consists of a light chain variable region ($V_L$) and a light chain constant region ($C_L$). The light chain constant region consists of one domain, $C_L$. The constant regions of the antibody can mediate the binding of the immunoglobulin to host tissues or factors, including various cells of the immune system (e.g., effector cells) and component C1q of the complement system. $V_H$ and $V_L$ can also be subdivided into highly variable regions called complementarity-determining regions (CDRs), interspersed with more conservative regions called framework regions (FRs). Each $V_H$ and $V_L$ consists of, from the amino terminus to the carboxyl terminus, three CDRs and four FRs arranged in the following order: FR1, CDR1, FR2, CDR2, FR3, CDR3, FR4. The variable regions ($V_H$ and $V_L$) of each heavy chain/light chain pair form an antibody binding site respectively. The assignment of amino acids to each region or structural domain follows the definition of Kabat Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883.

The monoclonal antibody variant described herein can be obtained through traditional genetic engineering methods. Those skilled in the art are fully aware of methods for modifying DNA molecules using nucleic acid mutations. In addition, nucleic acid molecules encoding heavy chain and light chain variants can also be obtained through chemical synthesis.

In the present invention, algorithms used to determine sequence identity (homology) and percent sequence similarity are, for example, the BLAST and BLAST 2.0 algorithms, respectively described by Altschul et al., (1977) Nucl. Acid. Res. 25: 3389-3402 and Altschul et al., (1990) J. Mol. Biol. 215: 403-410. BLAST and BLAST2.0 can be used to determine percent of amino acid sequence identity of the present invention using, for example, parameters described in the references or default parameters. Software used to perform BLAST analysis is publicly available through the National Center for Biotechnology Information.

As described herein, said amino acid sequence having at least 70% identity to an amino acid sequence includes a polypeptide sequence that is substantially identical to said amino acid sequence, e.g., those sequences having at least 70%, preferably at least 75%, 80%, 85%, 86%, 87%, 88%, 89%, 90%, 91%, 92%, 93%, 94%, 95%, 96%, 97%, 98%, 99% or higher identity to the polypeptide sequence of the invention when using the methods described herein (such as BLAST analysis using standard parameters).

The mutant of said amino acid sequence, as used herein, refers to a sequence which has identity of more than 70%, such as more than 75%, 80%, 85%, 90%, 91%, 92%, 93%, 94%, 95%, 96%, 97%, 98%, or 99%, to said amino acid sequence, such as the sequence having three, two or one substitution, deletion or addition of amino acids. Preferably, no more than three amino acids are substituted, added or deleted. More preferably, no more than two amino acids are substituted, added or deleted. Most preferably, no more than one amino acid is substituted, added or deleted.

A "substitutional" variant is one in which at least one amino acid residue in the native sequence has been removed and a different amino acid inserted in its same position. The substitutions can be single, wherein only one amino acid is substituted in the molecule, or multiple, wherein the same molecule has two or more amino acids substituted. Multiple substitutions can be made at consecutive sites. Likewise, one amino acid may be substituted by multiple residues, wherein such variants include both substitutions and insertions. An "insertion" (or "additive") variant is one in which one or more amino acids are inserted into a particular position immediately adjacent to a native sequence. Immediately adjacent to an amino acid means attachment to the alpha-carboxyl or alpha-amino functional group of the amino acid. A "deletion" variant is one in which one or more amino acids in the native amino acid sequence have been removed. Typically, deletion variants have one or two amino acids deleted in a specific region of their molecule.

In the present invention, the structure of the MMAE is

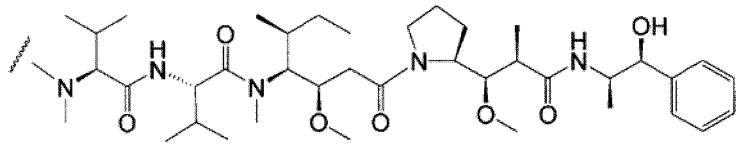

In the present invention, in formula I, L is MC-vc-PAB, D is MMAE, that is, in a case where L-D is MC-vc-PAB-MMAE, its structure is represented by:

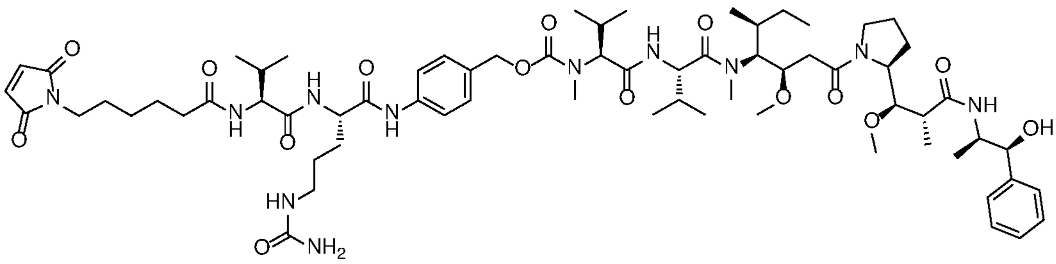

.

In the present invention, the anti-EGFR antibody Ab is linked to the L-D through thiol generated after its own disulfide bond is reduced. For example, in the case where L-D is MC-vc-PAB-MMAE, the structure after the Ab is linked to the L-D is represented by:

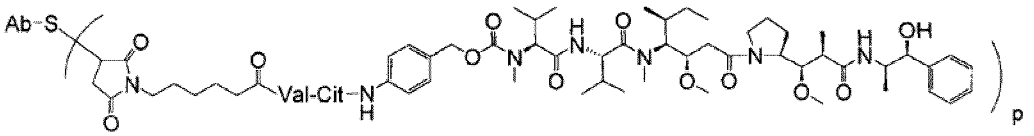

.

In the present invention, the drug antibody ratio (DAR) or drug load is represented by p, i.e., the average number of drug modules (i.e., cytotoxic agents) per antibody in the molecule of formula I: Ab-$(L-D)_p$, which may be an integer or a fraction. ADC of general formula I includes a collection of antibodies conjugated with a range of drug modules. The average number of drug modules per antibody in an ADC formulation from conjugation reaction can be verified by conventional means, such as mass spectrometry, ELISA, HIC and HPLC. The quantitative distribution of ADC in p can also be determined. In some cases, the separation, purification and verification of homogeneous ADC with p of a certain value from ADC with other DAR can be achieved by means such as reversed-phase HPLC or electrophoresis.

In certain embodiments, less than the theoretical maximum of the drug moiety is conjugated to the antibody in the conjugation reaction. In general, antibodies do not contain many free and reactive cysteine thiol groups that can link drug moieties; in fact, most cysteine thiol groups in antibodies exist as disulfide bridges. In certain embodiments, the antibody can be reduced with a reducing agent such as dithiothreitol (DTT) or tris(2-carboxyethyl)phosphine (TCEP) under partially or fully reducing conditions to generate reactive cysteine thiol groups.

In the present invention, "treatment" refers to a clinical intervention that attempts to alter the natural course of the individual or cell being treated, either for prevention or in the course of clinical pathology. The desired effects of treatment include preventing the occurrence or recurrence of disease, alleviating symptoms, attenuating any direct or indirect pathological consequences of the disease, preventing metastasis, slowing down the disease progression, ameliorating or alleviating the disease state, and eliminating or improving prognosis. In some embodiments, the antibody-drug conjugate of the present invention is used to delay the onset of a disease or disorder, or to slow down the progression of a disease or disorder. The above-described parameters for assessing successful treatment and amelioration of disease can be readily measured by routine procedures familiar to physicians. For cancer treatment, efficacy can be measured, for example, by assessing time to progression of disease (TTP) and/or determining response rate (RR).

In the present invention, the term "subject" refers to vertebrates. In some embodiments, the vertebrates are mammals. The mammals include, but are not limited to, livestock (such as cattle), pets (such as cats, dogs, and horses), primates, mice and rats. In some embodiments, the mammals refer to humans.

In the present invention, "effective amount" refers to an amount effective to achieve the desired therapeutic or prophylactic effect at the necessary dose and time. The "therapeutically effective amount" of a substance/molecule of the present invention may vary depending on factors such as the disease state, age, sex and weight of the individual and the ability of the substance/molecule to elicit a desired response in the individual. A therapeutically effective amount also encompasses an amount in which any toxic or detrimental consequences of the substance/molecule are outweighed by the therapeutically beneficial effects. The "prophylactically effective amount" refers to an amount effective at the necessary dose and time to achieve the desired prophylactic effect. Usually, but not necessarily, the prophylactically effective amount will be less than the therapeutically effective amount because the prophylactic dose is administered to the subject prior to the onset of the disease or at an early stage of the disease. In the case of cancer, the therapeutically effective amount of the drug reduces the number of cancer cells; shrinks the tumor size; inhibits (i.e., slows to some extent, preferably stops) infiltration of cancer cells into surrounding organs; inhibits (i.e., slows to some extent, preferably

stops) tumor metastasis; inhibits tumor growth in some degree; and/or alleviates one or more symptoms associated with cancer in some degree.

For the prevention or treatment of disease, the appropriate dosage of the antibody-drug conjugate of the present invention (when used alone or in combination with one or more other therapeutic agents such as chemotherapeutic agents) will depend on the type of disease to be treated, the type of the antibody-drug conjugate, severity and progression of the disease, whether the antibody-drug conjugate is administered for prophylactic or therapeutic purposes, previous therapies, the patient's clinical history and reactivity to the antibody-drug conjugate, and the attending physician's judgment. Suitably, the antibody-drug conjugate is administered to the patient either once or over a series of treatments.

In the present invention, the term "synergistic" refers to the observed consequences of producing such an effect (e.g., inhibition of tumor growth, prolonged survival time, etc.), in combined administration of components or agents (e.g., combined administration of an antibody-drug conjugate such as MYK-3 and an anti-PD-1 antibody such as AK103 or an anti-PD-L1 antibody), that is greater than the effect which would be expected based on the additive properties or effects of the individual components. In some embodiments, the synergistic effect is determined by performing a Bliss analysis (see, e.g., Foucquier et al., Pharmacol. Res. Perspect. (2015)3(3): e00149, which is incorporated herein by reference in its entirety for all purposes). The Bliss Independence model assumes that drug effect is the result of a probabilistic process and that drugs act completely independently (that is, drugs do not interfere with each other (for example, drugs have different action sites), but each contributes to a common result). According to the Bliss Independence model, the expected effect of a combination of two drugs is calculated using the following equation:

$$E_{AB} = E_A + E_B - E_A \times E_B,$$

wherein $E_A$ and $E_B$ represent the effects of drugs A and B respectively, while $E_{AB}$ represents the effect of the combination of drugs A and B. When the observed effect of the combination is higher than the expected effect $E_{AB}$, the combination of the two drugs is considered synergistic. When the observed effect of the combination is equal to $E_{AB}$, a combination of two drugs is considered additive. Alternatively, when the observed effect of the combination is lower than that of $E_{AB}$, a combination of two drugs is considered antagonistic.

The observed effect of a drug combination can be based on, for example, the TGI index of a subject or a population of subjects, tumor size (e.g., volume, mass), absolute change in tumor size (e.g., volume, mass) between two or more time points (e.g., between the first day of administration of treatment and a specified number of days after first administration of treatment), the rate of change in tumor size (e.g., volume, mass) between two or more time points (e.g., between the first day of administration of treatment and a specified number of days after first administration of treatment), or survival time. When the TGI index is used as a measure of an observed effect of a drug combination, the TGI index may be determined at one or more time points. When the TGI index is determined at two or more time points, in some cases the mean or median of multiple TGI indices may be used as a measure of the observed effect. Furthermore, the TGI index may be determined in a subject or in a population of subjects. When the TGI index is determined in a population, the mean or median TGI index in the population (e.g., at one or more time points) may be used as a measure of the observed effect. When tumor size or tumor growth rate is used as a measure of an observed effect, the tumor size or tumor growth rate may be measured in a subject or a population of subjects. In some cases, the mean or median tumor size or tumor growth rate is determined for a subject at two or more time points, or in a population of subjects at one or more time points. When survival time is determined in a population, the mean or median survival time may be used as a measure of an observed effect.

"Pharmaceutically acceptable carrier", as used herein, generally includes pharmaceutically acceptable carriers, excipients or stabilizers that are nontoxic for cells or mammals to which they are exposed at the doses and concentrations employed. Typically, the physiologically acceptable carriers refer to aqueous pH buffer solutions. Examples of physiologically acceptable carriers include buffers, such as phosphates, citrates, and other organic acids; antioxidants, including ascorbic acid; low-molecular-weight (less than about 10 residues) polypeptides; proteins, such as serum albumin, gelatin, or immunoglobulins; hydrophilic polymers, such as polyvinylpyrrolidone; amino acids, such as glycine, glutamine, asparagine, arginine or lysine; monosaccharides, disaccharides and other carbohydrates, including glucose, mannose, sucrose, trehalose or dextrin; chelating agents, such as EDTA; sugar alcohols, such as mannitol or sorbitol; salt-forming counterions, such as sodium; and/or nonionic surfactants, such as TWEEN™, polyethylene glycol (PEG) and PLURONICS™.

In some embodiments, the pharmaceutically acceptable salt is an inorganic acid salt or an organic acid salt, wherein the inorganic acid salt is hydrochloride, hydrobromide, hydroiodide, nitrate, bicarbonate, carbonate, sulfate or phosphate, the organic acid salt is formate, acetate, propionate, benzoate, maleate, fumarate, succinate, tartrate, citrate, ascorbate, α-ketoglutarate, α-glycerophosphate, alkyl sulfonate or aryl sulfonate; preferably, the alkyl sulfonate is methanesulfonate or ethanesulfonate; the aryl sulfonate is benzenesulfonate or p-toluene sulfonate.

Pharmaceutically acceptable salts can be obtained using standard procedures well known in the field, for example, by reacting a sufficient amount of a basic compound with a suitable acid which provides a pharmaceutically acceptable anion.

In the present invention, solvates refer to these forms of the antibody-drug conjugates of the present invention: complexes in solid or liquid form formed by coordination of the antibody-drug conjugates with solvent molecules. Hydrates are a specific form of solvates, which have coordinated water molecules. In the present invention, hydrates are the preferred solvates.

In the present invention, EGFR overexpression refers to an increase in the expression level of EGFR compared with the EGFR expression level on the surface of normal epithelial cells. It can be divided into high expression, medium expression and low expression (Wild, R., et al., Mol. Cancer Rher 2006:5(1), p104-113, Cetuximab preclinical antitumor activity (monotherapy and combination based) is not predicted by relative total or activated epidermal growth factor receptor tumor expression levels).

In the present invention, the 20 conventional amino acids and their abbreviations follow conventional usage. See Immunology-A Synthesis (second version, E.S. Golub and D.R. Gren, Eds., Sinauer Associates, Sunderland, Mass. (1991)), which is incorporated herein by reference.

MYK-3 is an antibody-drug conjugate (ADC), which is formed by conjugation of an anti-EGFR monoclonal antibody (BA03) and a potent cytotoxic small molecule drug MMAE (methyl auristatin E) via vc (i.e., 6-maleimidocaproyl-valine-citrulline-p-aminobenzyloxycarbonyl, also known as MC-vc-PAB) linker. By binding to the EGFR receptor on the surface of tumor cells, MYK-3 is endocytosed and releases MMAE, thereby blocking various cellular physiological functions involving tubulin, including mitosis, and further inhibiting tumor cell proliferation and leading to tumor cell death.

AK103 is an anti-PD-1 monoclonal antibody drug and an immune checkpoint inhibitor. By blocking the PD-1/PD-L1 signaling pathway, T cell activation is up-regulated and the endogenous anti-tumor immune response is activated, thereby exerting a therapeutic effect on tumors.

The antibody BA03 in the present invention is the BA03 in the Chinese invention patent application CN103772504A. For its preparation method, refer to Example 3 in the patent application. The sequences of each part of the antibody are as follows:

the sequence of the heavy chain variable region is:

QVQLQESGPGLVKPSETLSLTCTVSGFSLSNYDVHWVRQAPGKGLEWLGVIWSGGNTDYNTPFTSRLTISVDTSKNQFSLKLSSVTAADTAVYYCARALDYYDYEFAYWGQGTLVTVSS (SEQ ID NO：1).

In the sequence, the underlined parts are CDR1 (SEQ ID NO: 5), CDR2 (SEQ ID NO: 6), and CDR3 (SEQ ID NO: 7), respectively;
the parts without underline are FR1 (SEQ ID NO: 8), FR2 (SEQ ID NO: 9), FR3 (SEQ ID NO: 10), and FR4 (SEQ ID NO: 11), respectively.

The sequence of the light chain variable region is:

EIVLTQSPDFQSVTPKEKVTITCRASQSIGTNIHWYQQKPDQSPKLLIKYASESISGIPSRFSGSGSGTDFTLTINSLEAEDAATYYCQQNNEWPTSFGQGTKLEIK (SEQ ID NO: 2).

In the sequence, the underlined parts are CDR1 (SEQ ID NO: 12), CDR2 (SEQ ID NO: 13), and CDR3 (SEQ ID NO: 14), respectively;
the parts without underline are FR1 (SEQ ID NO: 15), FR2 (SEQ ID NO: 16), FR3 (SEQ ID NO: 17), and FR4 (SEQ ID NO: 18), respectively.

The sequence of the heavy chain constant region is:

ASTKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKRVEPKSCDKTHTCPPCPAPELLGGPSVFLFPPKPKDTL MISRTPEVTCVVVDVSHEDPEVKFNWYVDGVEVHNAKTKPREEQYNSTYRVVSVLTVLHQDWLNGK EYKCKVSNKALPAPIEKTISKAKGQPREPQVYTLPPSREEMTKNQVSLTCLVKGFYPSDIAVEWESNG QPENNYKTTPPVLDSDGSFFLYSKLTVDKSRWQQGNVFSCSVMHEALHNHYTQKSLSLSPGK (SEQ ID NO: 3).

The sequence of the light chain constant region is:

RTVAAPSVFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGEC (SEQ ID NO: 4).

The present invention will be further explained below with reference to specific examples, but these examples do not limit the scope of the present invention.

It should be noted that in the present invention and in the following examples, ADC-3 or MRG003 refers to MYK-3.

Example 1: Preparation method of antibody-drug conjugate

10 mg of antibody BA03 was buffer exchanged into a reducing buffer (25 mM sodium borate, pH 8.0, 25 mM NaCl, 5 mM EDTA) using a 15 mL 30KD ultrafiltration device for three times in total (the final volume was about 1 mL), transferred to a new Eppendorf centrifuge tube (weighed), and weighed. The concentration of the protein was detected and the total amount of the protein was calculated. 2.5 times molar amount of DTT was added to the antibody, the mixture was incubated at room temperature for 2 h and mixed continuously. The mixture was buffer exchanged into a coupling buffer (50 mM Tris, pH 7.2, 150 mM NaCl, 5 mM EDTA) using a 15 ml 30KD ultrafiltration device for three times in total. The concentrated solution was taken, measured for the concentration of the protein by A280, and weighed, and the total amount of the protein was calculated. 10 μl of sample was taken to measure the number of free thiol groups by Ellman's test;

and the molar concentration of its free thiol groups was calculated according to the following formula:

$$C_{thiol} = \frac{A412 \times 112}{b \times 14150} \ (M)$$

b: optical path length of cuvette (usually 1 cm).

The mole number of free thiol groups was calculated according to the molar concentration of free thiol groups and the volume of the total protein solution.

vc-MMAE (purchased from Shanghai Haoyuan Chemexpress Co., Ltd., Cat. No. HY-15575) (dissolved in DMSO), 1.1 times the mole number of free thiol groups, was added to the reduced antibody, and the mixture was well mixed to react at room temperature for 2 h, with intermittent mixing. N-acetylcysteine, 20 times the mole number of the vc-MMAE, was added to the reaction system. The reaction mixture was well mixed, and allowed to stand for 5 min. The mixture was buffer exchanged into the conjugate stock solution (20 mM Na-citrate, 0.3% NaCl, 5% Trehalose, 0.05% TWeen-80, pH 6.0) using a 15 ml 30KD ultrafiltration device for three times in total to obtain the antibody-drug conjugate MYK-3. The sample was stored at 4 °C.

Determination of drug-to-antibody ratio:

The prepared antibody-drug conjugate was analyzed by HIC-HPLC (Jun Ouyang, Drug-To-Antibody (DAR) Ratio and Drug Distribution by Hydrophobic Interaction Chromatography and Reverse Phase High Performance Chromatography, Laurent Ducry (ed.), Antibody Drug Conjugates, Chapter 17, Methods in Molecular Biology, Vol 1045, p275-283) to determine the drug-to-antibody ratio (DAR). As shown in Fig. 1, the average DAR calculated according to the peak area of the spectrum was 4.1.

Example 2 In vitro killing activity of MYK-3 against cell lines with EGFR mutations

In order to study the killing effect of MYK-3 against cell lines with EGFR mutations, two human lung cancer cell lines NCI-H1975 and NCI-H1650 with different EGFR mutations were selected, and the killing effects of MYK-3 and commercial reference drug Erbitux® were measured by the cell proliferation inhibition method using CCK-8 test reagent. The research results are shown in Table 1 and Figs. 2-3.

Table 1 Killing effects of MYK-3 and Erbitux® against two cell lines with different EGFR mutations

| Cell line | Tissue source | EGFR Expression level | Mutation site | $IC_{50}$ (Mean±SD, ng/mL) | |
|---|---|---|---|---|---|
| | | | | MYK-3 | Erbitux® |
| NCI-H1975 | NSCLC | Medium expression | L858R/T790M | 3.0±0.7 | ND |
| NCI-H1650 | Bronchoal veolar carcinoma | Medium expression | DelE746-A750 | $3.0*10^3 \pm 7.9*10^2$ | ND |

Note: For EGFR expression levels and mutation sites, please refer to reference documents. The value of $IC_{50}$ is the mean of the $IC_{50}$ calculated in two 96-well plates: ND indicates that the highest proliferation inhibition% is less than 50%, so the value of $IC_{50}$ cannot be calculated.

Experimental results show that MYK-3 has significant cell killing effects against two human lung cancer cell lines with different EGFR mutations, and the effects are significantly better than those of commercially available reference drug Erbitux®.

Example 3 Tumor inhibition effect of MYK-3 in CDX model

The human-derived tumor cell xenograft model (CDX model) is a tumor model established using human tumor cells in immunodeficient mice. In order to effectively evaluate the efficacy of MYK-3 in future clinical indications, experiments were conducted in an EGFR-mutated human NSCLC CDX model. In all experiments, the commercially available EGFR-targeted monoclonal antibody drug Erbitux® was used as a reference drug to compare tumor inhibition activities.

Table 2: Information of the PDX model used in pharmacodynamic experiments in vivo

| Model No. | EGFR expression | Mutation |
|---|---|---|
| NCI-H1975 | High expression | L858R/T790M |

Nude mice were subcutaneously inoculated with human lung cancer cell H1975. After the tumors grew to 100-200mm$^3$, the animals were randomly divided into groups (D0). MYK-3 was administered at doses of 0.3 mg/kg, 1 mg/kg and 3 mg/kg, and the tumor inhibition rates on day 14 (D14) were 34%, 128% and 141%, respectively. In the 1 mg/kg and 3 mg/kg dose groups, 7 out of 8 mice showed partial tumor regression. By day 26 (D25), the 1 mg/kg group still had 5 out of 8 mice with partial tumor regression, while the 3 mg/kg group had 5 out of 8 mice with partial tumor regression and 2 out of 8 mice with complete tumor regression. The tumor inhibition rate of the reference drug Erbitux® (3 mg/kg, IV) against H1975 was 82% (D14). The above drugs were well tolerated by tumor-bearing mice. The efficacy of ADC-3 administered at a dose of 1 mg/kg against H1975 was significantly better than that of Erbitux® administered at a dose of 3 mg/kg. The experimental results are shown in Fig. 4.

Example 4 Tumor inhibition effect of MYK-3 in PDX model

The human-derived tumor tissue xenograft model (PDX model) is a tumor model established in immunodeficient mice using human tumor tissue, which retains the heterogeneity, molecular diversity and histological characteristics of the primary tumor to the greatest extent. It has a high predictive value for the clinical therapeutic effect of drugs and has been increasingly used in cancer research (Hidalgo-2014) in recent years. In order to effectively evaluate the efficacy of MYK-3 in future clinical indications, experiments were conducted in five AZD9291-resistant human NSCLC PDX models. In all experiments, the commercially available third-generation EGFR inhibitor AZD9291 was used as a reference drug to compare tumor inhibition activities. The information of the five PDX models used in the study is shown in Table 3.

Table 3: Information of PDX models used in pharmacodynamic experiments in vivo

| Model No. | Gender | Age | Pathological diagnosis | Generation used in experiments | EGFR mRNA | Mutation site | AZD9291 resistance |
|---|---|---|---|---|---|---|---|
| LUN#2210-4a | Male | 53 | Adenosquamo us carcinoma of the lung | P2 | High expression | CDKN2A/2 B, STK11 | Yes |
| LUN#2210-106 | Male | 53 | Adenosquamo us carcinoma of the lung | P2 | High expression | L858R, c-Met amplification | Yes |
| LUN#2355-128 | Male | 49 | Large cell lung carcinoma | P2 | High expression | L858R/T790M | Yes |
| LUN#2441-118 | Female | 49 | Adenosquamo us carcinoma of the lung | P2 | Medium expression | Not available | Yes |
| LUN#2005-143 | Male | 60 | Lung adenocarcino ma | P2 | High expression | L858R, HER3 amplification | Yes |

1) Study on the efficacy of MYK-3 in the AZD9291-resistant human lung cancer PDX model LUN#2005-143

LUN#2005-143 is an AZD9291-resistant human lung adenocarcinoma (a type of NSCLC) PDX model. This experiment was performed in total of 4 groups, including a Vehicle group (ADC-3 vehicle), an AZD9291 administration group (5 mg/kg), and ADC-3 administration groups (1 mg/kg and 3 mg/kg). During the entire experiment, ADC-3 was administered four times in total, via tail vein injection on Day 0, Day 4, Day 8, and Day 12, and AZD9291 was administered 29 times in total, once a day by intragastric administration. The experimental results are shown in Fig. 5. On Day 29, the relative tumor proliferation rate T/C (%) for the AZD9291 (5 mg/kg) administration group was 36.90% (P<0.0001), with a tumor growth inhibition rate TGI% of 63.10%; the T/C (%) for the ADC-3 (1 mg/kg) administration group was 69.86% (P<0.001), with a TGI% of 30.14%; the T/C (%) for the ADC-3 (3 mg/kg) administration group was 25.27% (P<0.0001), with a TGI% of 74.73%. On Day 29, in the ADC-3 (3 mg/kg) group, complete tumor regression was observed in 1 out of 8 animals. In terms of body weight, all animals in the tested drug groups had good tolerance. Experimental results showed that AZD9291 (5 mg/kg) and ADC-3 (3 mg/kg) both could significantly inhibit tumor growth, and ADC-3 (3 mg/kg) had a better inhibition effect on tumors than AZD9291 (5 mg/kg); ADC-3 (1 mg/kg) showed certain anti-tumor activity, but had no significant inhibition effect on tumors [T/C (%) > 40%]. In this experiment, tumor-bearing mice had good tolerance to the tested drugs.

2) Study on the efficacy of MYK-3 in the AZD9291-resistant human lung cancer PDX model LUN#2210-4a

LUN#2210-4a is an AZD9291-resistant human adenosquamous carcinoma of the lung PDX model. This experiment was performed in total of 4 groups, including a Vehicle group (ADC-3 vehicle), an AZD9291 administration group (5 mg/kg), and ADC-3 administration groups (1 mg/kg and 3 mg/kg). During the entire experiment, ADC-3 was administered four times in total, via tail vein injection on Day 0, Day 4, Day 8, and Day 12, and AZD9291 was administered 28 times in total, once a day by intragastric administration. The experimental results are shown in Fig. 6. On Day 28, the T/C (%) for the AZD9291 (5 mg/kg) administration group was 68.99% (P<0.0001), with a TGI% of 31.01%; the T/C (%) for the ADC-3 (1 mg/kg) administration group was 13.10% (P<0.0001), with a TGI% of 86.90%; the T/C (%) for the ADC-3 (3 mg/kg) administration group was 0.31% (P<0.0001), with a TGI% of 99.69%. On Day 28, in the ADC-3 (1 mg/kg) and ADC-3 (3 mg/kg) administration groups, complete tumor regression was observed in 1 out of 8 animals and 7 out of 8 animals, respectively. Tumor-bearing mice had good tolerance to the tested drugs.

3) Study on the efficacy of MYK-3 in the AZD9291-resistant human lung cancer PDX model LUN#2210-106

LUN#2210-106 is an AZD9291-resistant human adenosquamous carcinoma of the lung PDX model. This experiment was performed in total of 4 groups, including a Vehicle group (ADC-3 vehicle), an AZD9291 administration group (5 mg/kg), and ADC-3 administration groups (1 mg/kg and 3 mg/kg). During the entire experiment, ADC-3 was

administered four times in total, via tail vein injection on Day 0, Day 4, Day 8, and Day 12, and AZD9291 was administered 30 times in total, once a day by intragastric administration. The experimental results are shown in Fig. 7. On Day 30, the T/C (%) for the AZD9291 (5 mg/kg) administration group was 65.31% ($P<0.0001$), with a TGI% of 34.69%; the T/C (%) for the ADC-3 (1 mg/kg) administration group was 55.49% ($P<0.0001$), with a TGI% of 44.51%; the T/C (%) for the ADC-3 (3 mg/kg) administration group was 0.0% ($P<0.0001$), with a TGI% of 100.00%. On Day 30, in the ADC-3 (3 mg/kg) administration group, complete tumor regression was observed in 1 out of 8 animals. As can be seen from the experimental results, ADC-3 (3mg/kg) could significantly inhibit tumor growth during the entire experiment period. ADC-3 (1 mg/kg) could significantly inhibit tumor growth during the administration period; after stopping the administration following 4 doses (Day 12) until the end of the experiment (Day 30), although there was some anti-tumor activity, there was no significant inhibition effect on the tumor [T/C (%) > 40%]. AZD9291 (5mg/kg) showed certain anti-tumor activity during the entire experiment period but had no significant inhibition effect on tumors [T/C (%)>40%]. Tumor-bearing mice had good tolerance to the tested drugs.

4) Study on the efficacy of MYK-3 in the AZD9291-resistant human lung cancer PDX model LUN#2355-128

LUN#2355-128 is an AZD9291-resistant human large cell lung carcinoma PDX model. This experiment was performed in total of 4 groups, including a Vehicle group (ADC-3 vehicle), an AZD9291 administration group (5 mg/kg), and ADC-3 administration groups (1 mg/kg and 3 mg/kg). During the entire experiment, ADC-3 was administered four times in total, via tail vein injection on Day 0, Day 4, Day 8, and Day 12, and AZD9291 was administered 27 times in total, once a day by intragastric administration. The experiment period was 27 days. The experimental results are shown in Fig. 8. On Day 16, the T/C (%) for the AZD9291 (5 mg/kg) administration group was 33.61% ($P<0.0001$), with a TGI% of 66.39%; the T/C (%) for the ADC-3 (1 mg/kg) administration group was 45.32% ($P<0.0001$), with a TGI% of 54.68%; the T/C (%) for the ADC-3 (3 mg/kg) administration group was 3.60% ($P<0.0001$), with a TGI% of 96.40%, and complete tumor regression was observed in 1 out of 8 animals. On Day 27, complete tumor regression was observed in 1 out of 8 animals in the ADC-3 (3 mg/kg) administration group. Experimental results showed that AZD9291 (5 mg/kg) and ADC-3 (3 mg/kg) both could significantly inhibit tumor growth, and ADC-3 (3 mg/kg) had a better inhibition effect on tumors than AZD9291 (5 mg/kg); ADC-3 (1 mg/kg) showed certain anti-tumor activity but had no significant inhibition effect on tumors [T/C (%) > 40%]. Tumor-bearing mice had good tolerance to the tested drugs.

5) Study on the efficacy of MYK-3 in the AZD9291-resistant human lung cancer PDX model LUN#2441-118

LUN#2441-118 is an AZD9291-resistant human adenosquamous carcinoma of the lung PDX model. This experiment was performed in total of 4 groups, including a Vehicle group (ADC-3 vehicle), an AZD9291 administration group (5 mg/kg), and ADC-3 administration groups (1 mg/kg and 3 mg/kg). During the entire experiment, ADC-3 was administered four times in total, via tail vein injection on Day 0, Day 4, Day 8, and Day 12, and AZD9291 was administered 28 times in total, once a day by intragastric administration. The experiment period was 28 days. The experimental results are shown in Fig. 9. On Day 25, the T/C (%) for the AZD9291 (5 mg/kg) administration group was 29.35% ($P<0.0001$), with a TGI% of 70.65%; the T/C(%) for the ADC-3 (1 mg/kg) administration group was 5.68% ($P<0.0001$), with a TGI% of 94.32%; the T/C (%) for the ADC-3 (3 mg/kg) administration group was 3.44% ($P<0.0001$), with a TGI% of 96.56%. Experimental results showed that AZD9291 (5 mg/kg), ADC-3(1 mg/kg) and ADC-3 (3 mg/kg) all could significantly inhibit tumor growth, and ADC-3 (1 mg/kg) and ADC-3 (3 mg/kg) had significantly better anti-tumor activity than AZD9291 (5 mg/kg) ($P<0.05$). Tumor-bearing mice had good tolerance to the tested drugs.

6) Study on the efficacy of MYK-3 and AK103, as well as combination of MYK-3 and AK103 in human lung cancer PDX model LUN#2210-106 in Hu-HSC-NPG mice

LUN#2210-106 is an AZD9291-resistant human adenosquamous carcinoma of the lung PDX model. This experiment was performed in total of 4 groups, including a Vehicle group (ADC-3 vehicle), an ADC-3 administration group (1 mg/kg), an AK103 administration group (5 mg/kg), and an ADC-3 (1 mg/kg) + AK103 (5 mg/kg) administration group. During the entire experiment, ADC-3 was administered three times in total for the ADC-3 single-drug administration group, via tail vein injection on Day 0, Day 4, and Day 8; and AK103 was administered every three days via tail vein injection for the AK103 single-drug administration group, 8 times in total. For the ADC-3 + AK103 administration group, ADC-3 was administered three times, and AK103 was administered ten times in total, and their administration modes were the same as those for the single-drug administration groups. The experiment period was 34 days. The experimental results are shown in Fig. 10. On Day 20, the T/C (%) for the ADC-3 (1 mg/kg) administration group was 13.31% ($P<0.05$), with a TGI% of 86.69%; the T/C (%) for the AK103 (5 mg/kg) administration group was 93.61% ($P>0.05$), with a TGI% of 6.39%; the T/C (%) for the ADC-3 (1 mg/kg) + AK103 (5 mg/kg) administration group was 2.77% ($P<0.005$), with a TGI% of 97.23%, and complete tumor regression was observed in 3 out of 6 animals. During the period from Day 20 to

Day 34, the mean tumor volume of animals in the ADC-3 (1 mg/kg) administration group gradually resumed growth (from 184 $mm^3$ to 893 $mm^3$); the mean tumor volume of animals in the ADC-3 (1 mg/kg) + AK103 (5 mg/kg) administration group grew slowly (from 38 $mm^3$ to 126 $mm^3$), and on Day 34, complete tumor regression was still observed in 1 out of 6 animals.

Whether the combination of ADC-3 and AK103 had a synergistic effect was calculated according to the equation $E_{AB} = E_A + E_B - E_A \times E_B$ and the calculation object was TGI%. Expected TGI% = 0.8669 + 0.0639-0.8669 $\times$ 0.0639=87.54%. Obviously, the expected TGI% (87.54%) was less than the observed TGI% (97.23%). Therefore, it could be determined that the combination of ADC-3 and AK103 had a synergistic effect.

The above results showed that compared with the vehicle control group, both ADC-3 (1 mg/kg) and the combination of ADC-3 (1 mg/kg) and AK103 (5 mg/kg) could significantly inhibit tumor growth, while AK103 (5 mg/kg) had no obvious inhibition effect on tumor growth. Compared with the administration of ADC-3 (1 mg/kg) alone, the same dose of ADC-3 combined with AK103 (5 mg/kg) had a more significant anti-tumor effect (Day 20, $P < 0.05$) and showed a synergistic effect. Tumor-bearing mice had good tolerance to the tested drugs.

The results of in vivo efficacy tests of MYK-3 in five AZD9291-resistant human lung cancer PDX models are summarized in Table 4.

Table 4: In vivo efficacy results of MYK-3 (i.v., q4d×4) in AZD9291-resistant human lung cancer PDX models

| Efficacy model | Tested drug | Dose (mg/kg) | Calculation date | Relative tumor proliferation rate T/C (%) | Tumor growth inhibition rate TGI% | Tumor regression condition: Partial regression | Tumor regression condition: Complete regression | Tumor inhibition activity |
|---|---|---|---|---|---|---|---|---|
| LUN#2005-143 | Vehicle (MYK-3 vehicle) | / | Day 29 | 100 | 0 | / | / | / |
| | MYK-3 | 1 | | 69.86 (P<0.001) | 30.14 | / | / | + |
| | | 3 | | 25.27 (P<0.0001) | 74.73 | / | 1/8 | +++ |
| | AZD9291 | 5 | | 36.90 (P<0.0001) | 63.10 | / | / | ++ |
| LUN#2210-4a | Vehicle (MYK-3 vehicle) | / | Day 28 | 100 | 0 | / | / | / |
| | MYK-3 | 1 | | 13.10 (P<0.0001) | 86.90 | / | 1/8 | +++ |
| | | 3 | | 0.31 (P<0.0001) | 99.69 | / | 7/8 | ++++ |
| | AZD9291 | 3 | | 68.99 (P<0.0001) | 31.01 | / | / | + |
| LUN#2210-106 | Vehicle (MYK-3 vehicle) | / | Day 30 | 100 | 0 | / | / | / |
| | MYK-3 | 1 | | 55.49 (P<0.0001) | 44.51 | / | / | + |

| Efficacy model | Tested drug | Dose (mg/kg) | Calculation date | Relative tumor proliferation rate T/C (%) | Tumor growth inhibition rate TGI% | Tumor regression condition: Partial regression | Tumor regression condition: Complete regression | Tumor inhibition activity |
|---|---|---|---|---|---|---|---|---|
| | | 3 | | 0.0 (P<0.0001) | 100.00 | / | 1/8 | ++++ |
| | AZD9291 | 5 | | 65.31 P<0.0001 | 34.69 | / | / | + |
| LUN#2355-128 | Vehicle (MYK-3 vehicle) | / | Day 16 | 100 | 0 | / | / | / |
| LUN#2355-128 | MYK-3 | 1 | Day 16 | 45.32 P<0.0001 | 54.68 | / | / | + |
| LUN#2355-128 | MYK-3 | 3 | Day 16 | 3.60 (P<0.0001) | 96.40 | / | 1/8 | ++++ |
| LUN#2355-128 | AZD9291 | 5 | Day 16 | 33.61 (P<0.0001) | 66.39 | / | / | ++ |
| LUN#2441-118 | Vehicle (MYK-3 vehicle) | / | Day 25 | 100 | 0 | / | / | / |
| LUN#2441-118 | MYK-3 | 1 | Day 25 | 5.68 (P<0.0001) | 94.32 | / | / | ++++ |
| LUN#2441-118 | MYK-3 | 3 | Day 25 | 3.44 (P<0.0001) | 96.56 | / | / | ++++ |
| LUN#2441-118 | AZD9291 | 3 | Day 25 | 29.35 (P<0.0001) | 70.65 | / | / | ++ |

Note: "/" means not applicable. The formula for calculating tumor volume (TV) is: TV= $l \times w^2/2$, wherein l and w represent the measured length and width of the tumor, respectively. Based on the measurement results, the relative tumor volume (RTV) is calculated with RTV=$V_f$ /V0, wherein V0 is the tumor volume measured at the time of group dosing (i.e., Day 0), and $V_f$ is the tumor volume measured on the last day. T/C (%)=(RTV of administration group/RTV of Vehicle group)×100%. TGI% = (mean tumor volume of Vehicle group - mean tumor volume of administration group)/mean tumor volume of Vehicle group × 100%. "++++" indicates T/C (%) ≥0 and ≤10%. "+++" indicates T/C (%)>10% and ≤20%. "++" indicates T/C (%) >20% and ≤40%. "+" indicates

T/C (%)>40%. The tumor inhibition activity of the tested drugs is classified based on the T/C (%) at the highest dose in the models.

The results of the in vivo efficacy tests of MYK-3 combined with the anti-PD-1 antibody in the AZD9291-resistant human lung cancer PDX models are summarized in Table 5.

Table 5: In vivo efficacy results of MYK-3(i.v., q4d×3) and AK103, as well as the combination of MYK-3 and AK103 in AZD9291-resistant human lung cancer PDX model

| Efficacy model | Tested drug | Dose (mg/kg) | Calculation date | Relative tumor proliferation rate T/C (%) | Tumor growth inhibition rate TGI% | Tumor regression condition: Partial regression | Tumor regression condition: Complete regression | Tumor inhibition activity |
|---|---|---|---|---|---|---|---|---|
| LUN#2210-106 | Vehicle (MYK-3 vehicle) | / | Day 20 | 100 | 0 | / | / | / |
| | MYK-3 | 1 | | 13.31 (P<0.005) | 86.69 | / | / | +++ |
| | AK103 | 5 | | 93.61 (P>0.005) | 6.39 | | | + |
| | MYK-3<br>AK103 | 1<br>5 | | 2.77 (P<0.005) | 97.23 | / | 3/6 | ++++ |

Note: "/" means not applicable. The formula for calculating tumor volume (TV) is: TV= l×w$^2$/2, wherein l and w represent the measured length and width of the tumor, respectively. Based on the measurement results, the relative tumor volume (RTV) is calculated with RTV=$V_f$ /V0, wherein V0 is the tumor volume measured at the time of group dosing (i.e. Day 0), and $V_f$ is the tumor volume measured on the last day. T/C (%)=(RTV of administration group/RTV of Vehicle group)×100%. TGI% = (mean tumor volume of Vehicle group - mean tumor volume of administration group)/mean tumor volume of Vehicle group × 100%. "++++" indicates T/C (%) ≥0 and ≤10%. "+++" indicates T/C (%)>10% and ≤20%. "++" indicates T/C (%) >20% and ≤40%. "+" indicates T/C (%)>40%. The tumor inhibition activity of the tested drugs is classified based on the T/C (%) at the highest dose in the models.

In vivo efficacy test results showed that in five AZD9291-resistant PDX models (all the models were NSCLC), MYK-3 showed significant tumor growth inhibition effect, and tumor-bearing mice had good tolerance to MYK-3. It should be noted that these five PDX models all had medium-high expression of EGFR and were resistant to AZD9291, and two

of them were resistant to EGFR mutations.

In the EGFR-mutated AZD9291-resistant PDX models, the combination of MYK-3 and AK103 (anti-PD-1 antibody) showed a more significant tumor growth inhibition effect than the same dose of single drug (MYK-3 or PD-1), achieving a synergistic effect. Moreover, after discontinuing administration until the end of the experiment, the mean tumor volume of the animals grew slowly, and complete tumor regression was still observed in 1 out of 6 animals. Compared to the dosage required to achieve therapeutic efficacy in monotherapy, the combination therapy was administered at a lower dose of MYK-3 or PD-1.

## Claims

**1.** Use of an antibody-drug conjugate, a pharmaceutically acceptable salt or solvate of the antibody-drug conjugate or a solvate of the salt, which is one or more selected from the group consisting of:

1) in the preparation of a non-small cell lung cancer inhibitor; and
2) in the preparation of a medicament for treating and/or preventing non-small cell lung cancer;

wherein, the non-small cell lung cancer is one or more selected from the group consisting of:

1) EGFR-TKI-resistant non-small cell lung cancer; and
2) EGFR mutated non-small cell lung cancer, and optionally, the EGFR mutated non-small cell lung cancer also has one or more selected from the group consisting of: CDKN2A/2B mutation, STK11 mutation, c-Met amplification, and HER3 amplification;

wherein, the antibody-drug conjugate has a structure of formula I,

$$Ab\text{-}(L\text{-}D)_p \qquad \text{formula I}$$

wherein:

Ab represents an anti-EGFR antibody, wherein the anti-EGFR antibody comprises a heavy chain and a light chain, wherein CDR1, CDR2, and CDR3 in a heavy chain variable region respectively comprise sequences as shown in SEQ ID NOs: 5-7 or mutants thereof, and CDR1, CDR2, and CDR3 in a light chain variable region respectively comprise sequences as shown in SEQ ID NOs: 12-14 or mutants thereof;
L represents a linker;
preferably, the linker is selected from the group consisting of 6-maleimidocaproyl (MC), maleimidopropionyl (MP), N-succinimidyl 4-(2-pyridylthio) valerate (SPP), 4-(N-maleimidomethyl)-cyclohexan-1-formyl (MCC), N-succinimidyl(4-iodo-acetyl)aminobenzoate (SIAB), and 6-maleimidocaproyl-valine-citrulline-p-aminobenzyloxycarbonyl (MC-vc-PAB);
more preferably, the linker is 6-maleimidocaproyl-valine-citrulline-p-aminobenzyloxycarbonyl (MC-vc-PAB);
D represents a cytotoxic agent;
preferably, the cytotoxic agent is selected from the group consisting of toxins such as SN-38, Gemcitabine, Monomethyl auristatin E (MMAE), Monomethyl auristatin F (MMAF), maytansinoids (such as Maytansine DM1 and Maytansine DM4), calicheamicin, MGBA (such as duocarmycin), doxorubicin, Ricin and diphtheria toxin, I131, interleukins, tumor necrosis factors, chemokines and nanoparticles;
more preferably, the cytotoxic agent is MMAE;
p represents 1-9, preferably 2-6, more preferably 3-5.

**2.** The use according to claim 1, wherein the anti-EGFR antibody has one or more of the following features:

1) FR1, FR2, FR3, and FR4 regions in the heavy chain variable region of the anti-EGFR antibody respectively comprise sequences as shown in SEQ ID NOs: 8-11 or mutants thereof;
2) FR1, FR2, FR3, and FR4 regions in the light chain variable region of the anti-EGFR antibody respectively comprise sequences as shown in SEQ ID NOs: 15-18 or mutants thereof;
3) the heavy chain constant region of the anti-EGFR antibody is selected from human IgG, IgM, IgA, IgD, and IgA constant regions or mutants thereof;
preferably, the IgG is selected from IgG1, IgG2, IgG3 and IgG4; and
4) the light chain constant region of the anti-EGFR antibody is selected from human lambda and kappa constant

regions or mutants thereof.

**3.** The use according to any one of claims 1-2, wherein the anti-EGFR antibody has one or more of the following features:

1) the sequence of the heavy chain variable region of the anti-EGFR antibody comprises a sequence as shown in SEQ ID NO: 1, or a sequence having greater than 70%, preferably greater than 75%, 80%, 85%, 90%, 95%, or 99% identity to the sequence as shown in SEQ ID NO: 1;
preferably, the sequence of the heavy chain variable region of the anti-EGFR antibody is shown in SEQ ID NO: 1;
2) the sequence of the light chain variable region of the anti-EGFR antibody comprises a sequence as shown in SEQ ID NO: 2, or a sequence having greater than 70%, preferably greater than 75%, 80%, 85%, 90%, 95%, or 99% identity to the sequence as shown in SEQ ID NO: 2;
preferably, the sequence of the light chain variable region of the anti-EGFR antibody is shown in SEQ ID NO: 2;
3) the sequence of the heavy chain constant region of the anti-EGFR antibody comprises a sequence as shown in SEQ ID NO: 3, or a sequence having greater than 70%, preferably greater than 75%, 80%, 85%, 90%, 95%, or 99% identity to the sequence as shown in SEQ ID NO: 3;
preferably, the sequence of the heavy chain constant region of the anti-EGFR antibody is shown in SEQ ID NO: 3; and
4) the sequence of the light chain constant region of the anti-EGFR antibody comprises a sequence as shown in SEQ ID NO: 4, or a sequence having greater than 70%, preferably greater than 75%, 80%, 85%, 90%, 95%, or 99% identity to the sequence as shown in SEQ ID NO: 4;
preferably, the sequence of the light chain constant region of the anti-EGFR antibody is shown in SEQ ID NO: 4.

**4.** The use according to any one of claims 1-3, wherein the EGFR-TKI-resistant non-small cell lung cancer has one or more selected from the group consisting of: EGFR mutation, CDKN2A/2B mutation, STK11 mutation, c-Met amplification, and HER3 amplification;

preferably, the EGFR mutation is a mutation at one or more sites selected from the group consisting of: exon 18, exon 19, exon 20, and exon 21;
more preferably, the EGFR mutation is one or more mutations selected from the group consisting of: DelE746-A750, L858R, T790M, C797S, G719S, L861Q, G719C, G719A, T854A, and D761Y.
further preferably, the EGFR mutation is one or more mutations selected from the group consisting of: DelE746-A750, L858R, and T790M;
most preferably, the EGFR mutation is DelE746-A750, L858R, T790M or L858R/T790M double mutation.

**5.** The use according to any one of claims 1-4, wherein the EGFR-TKI is one or more selected from the group consisting of: osimertinib (AZD9291), gefitinib, erlotinib, icotinib, afatinib, dacotinib, imatinib, lapatinib, nazartinib, rociletinib, naquotinib, vandetanib, neratinib, pelitinib, canertinib, brigatinib, PKC412, Go6976, mavelertinib, olmutinib, WZ4002, TAS2913, cetuximab, panitumumab, avitinib, HS-10296, and TQB3804;

preferably, the EGFR-TKI is osimertinib (AZD9291);
or, the non-small cell lung cancer is selected from the group consisting of lung adenocarcinoma (preferably, bronchoalveolar carcinoma), lung squamous cell carcinoma, adenosquamous carcinoma of the lung, and large cell lung carcinoma.

**6.** A combination drug, comprising:

a first drug, the first drug being an antibody-drug conjugate, a pharmaceutically acceptable salt or solvate of the antibody-drug conjugate, or a solvate of the salt; and
a second drug, the second drug being a PD-1/PD-L1 inhibitor;
wherein the antibody-drug conjugate is defined as in any one of claims 1-3;
preferably, the first drug and the second drug are separated from each other.

**7.** The combination drug according to claim 6, wherein the PD-1/PD-L1 inhibitor is an anti-PD-1 antibody or an anti-PD-L1 antibody;

preferably, the anti-PD-1 antibody is one or more selected from the group consisting of: AK103, Toripalimab (JS-001), Sintilimab (IBI308), Camrelizumab, Tislelizumab (BGB-A317), Opdivo or Nivolumab, Keytruda or Pembrolizumab, CS1003, serplulimab (HLX10), AK104, geptanolimab (GB226), lizumab (e.g., LZM009), BAT-1306, SCT-I10A, F520, SG001, GLS-010, PDR001, REGN2810, and STI-A1110;
more preferably, the anti-PD-1 antibody is AK103;
preferably, the anti-PD-L1 antibody is Durvalumab, Atezolizumab or a combination thereof.

**8.** The combination drug according to any one of claims 6-7, wherein the weight ratio of the first drug to the second drug is 15:1-1:15;
preferably, the weight ratio of the first drug to the second drug is 1:1-1:10.

**9.** Use of the combination drug according to any one of claims 6-8, which is one or more selected from the group consisting of:

1) in the preparation of a non-small cell lung cancer inhibitor; and
2) in the preparation of a medicament for treating and/or preventing non-small cell lung cancer;

wherein, the non-small cell lung cancer is one or more selected from the group consisting of:

1) EGFR-TKI-resistant non-small cell lung cancer; and
2) EGFR mutated non-small cell lung cancer, and optionally, the EGFR mutated non-small cell lung cancer also has one or more selected from the group consisting of: CDKN2A/2B mutation, STK11 mutation, c-Met amplification, and HER3 amplification.

**10.** The use according to claim 9, wherein the EGFR-TKI-resistant non-small cell lung cancer has one or more selected from the group consisting of: EGFR mutation, CDKN2A/2B mutation, STK11 mutation, c-Met amplification, and HER3 amplification;

preferably, the EGFR mutation is a mutation at one or more sites selected from the group consisting of: exon 18, exon 19, exon 20, and exon 21;
more preferably, the EGFR mutation is one or more mutations selected from the group consisting of: DelE746-A750, L858R, T790M, C797S, G719S, L861Q, G719C, G719A, T854A, and D761Y;
further preferably, the EGFR mutation is one or more mutations selected from the group consisting of: DelE746-A750, L858R, and T790M;
most preferably, the EGFR mutation is DelE746-A750, L858R, T790M or L858R/T790M double mutation;
or, the EGFR-TKI is one or more selected from the group consisting of: osimertinib (AZD9291), gefitinib, erlotinib, icotinib, afatinib, dacotinib, imatinib, lapatinib, nazartinib, rociletinib, naquotinib, vandetanib, neratinib, pelitinib, canertinib, brigatinib, PKC412, Go6976, mavelertinib, olmutinib, WZ4002, TAS2913, cetuximab, panitumumab, avitinib, HS-10296, and TQB3804;
preferably, the EGFR-TKI is osimertinib (AZD9291);
or, the non-small cell lung cancer is selected from the group consisting of lung adenocarcinoma (preferably, bronchoalveolar carcinoma), lung squamous cell carcinoma, adenosquamous carcinoma of the lung, and large cell lung carcinoma.

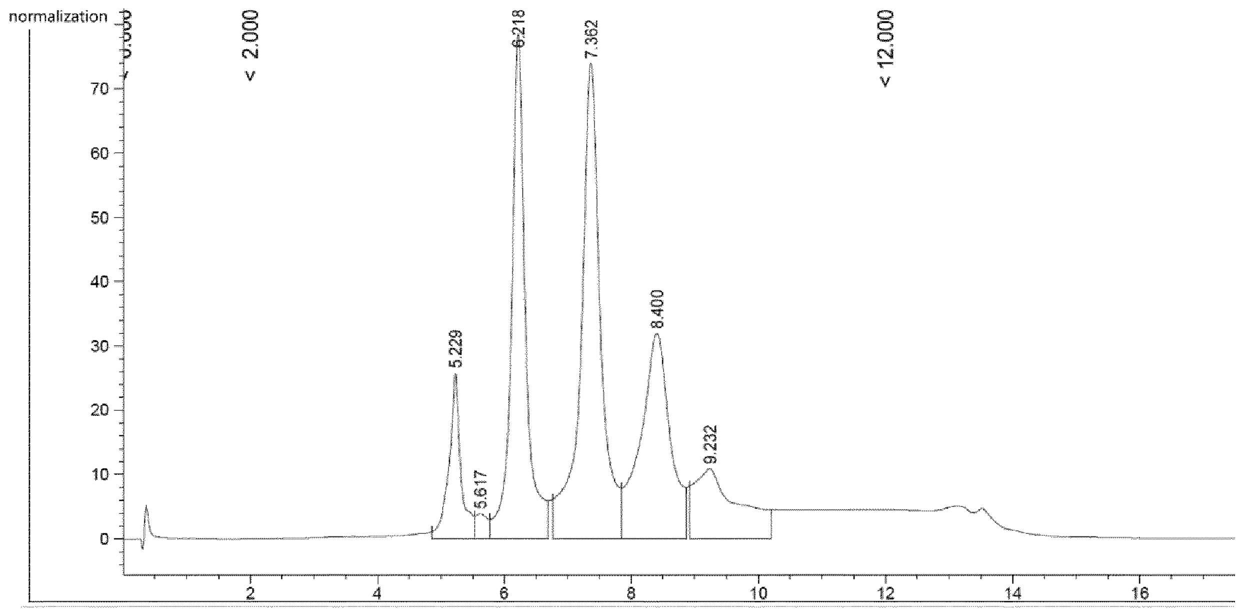
normalization
0.000
< 2.000
6.218
7.362
< 12.000
5.229
5.617
8.400
9.232
70
60
50
40
30
20
10
0
2
4
6
8
10
12
14
16

Fig. 1

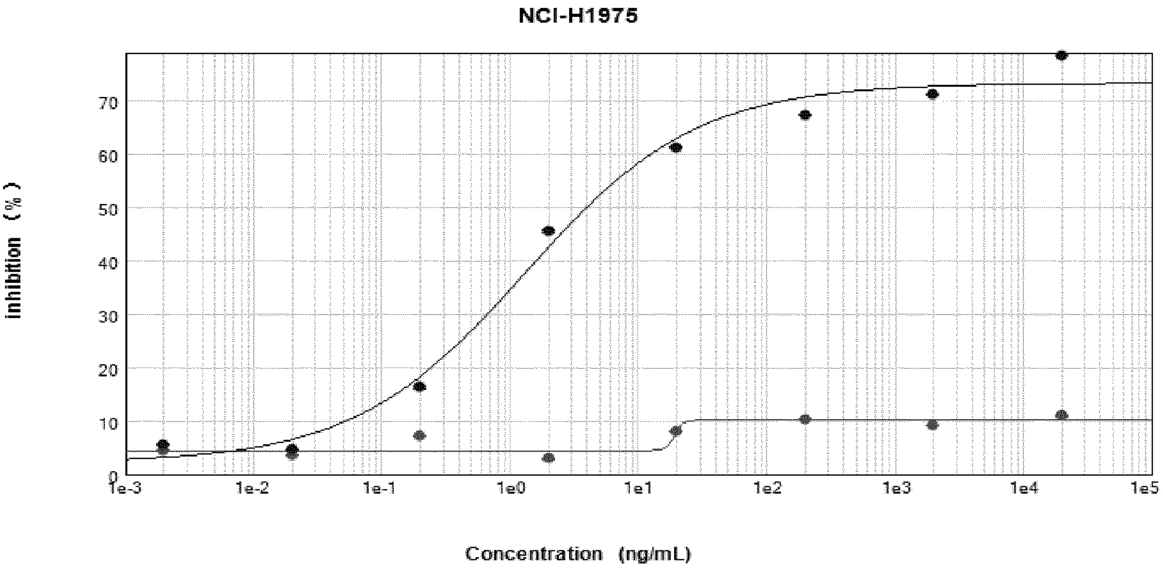
NCI-H1975
inhibition (%)
70
60
50
40
30
20
10
0
1e-3
1e-2
1e-1
1e0
1e1
1e2
1e3
1e4
1e5
Concentration (ng/mL)

Fig. 2

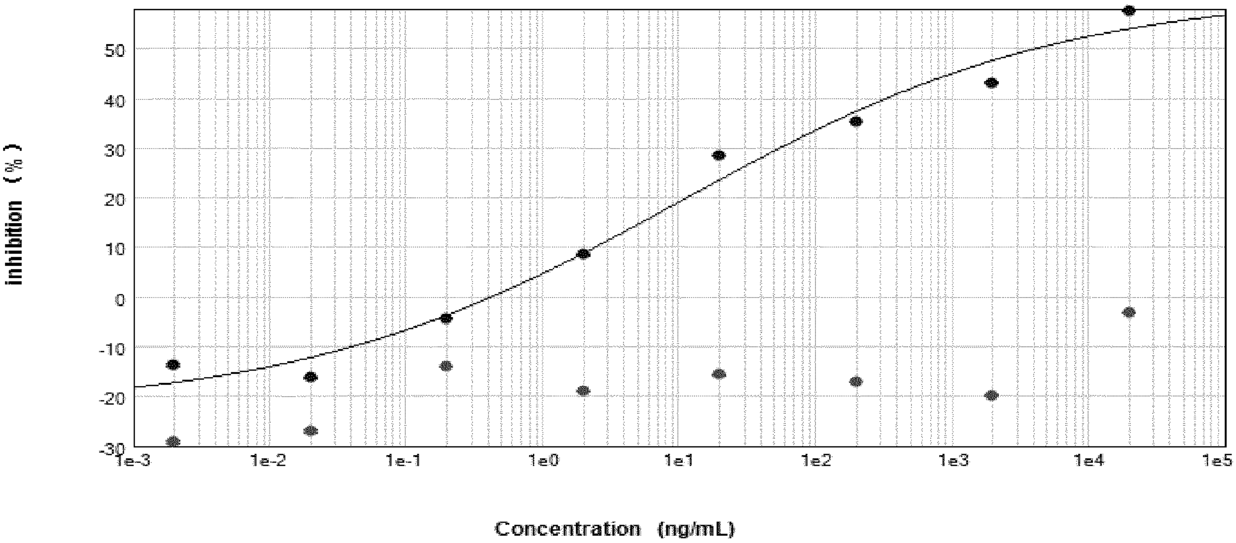
inhibition （%）
50
40
30
20
10
0
-10
-20
-30
1e-3
1e-2
1e-1
1e0
1e1
1e2
1e3
1e4
1e5
Concentration (ng/mL)

Fig. 3

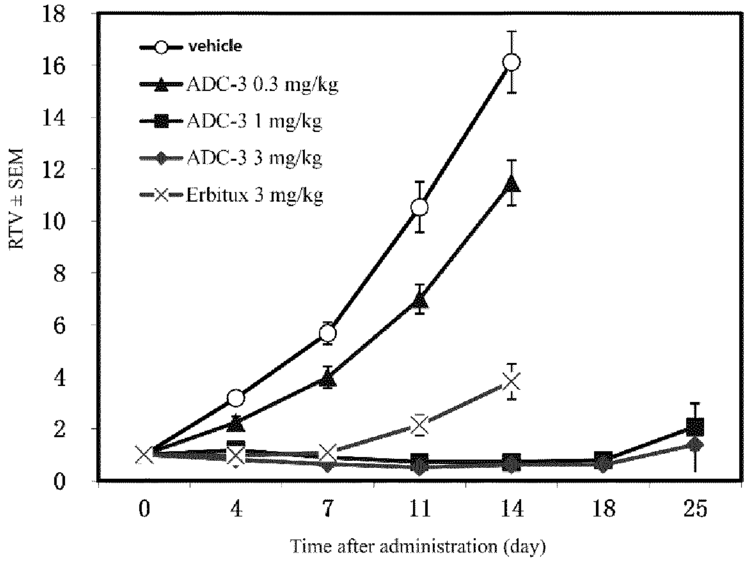
vehicle
ADC-3 0.3 mg/kg
ADC-3 1 mg/kg
ADC-3 3 mg/kg
Erbitux 3 mg/kg
RTV ± SEM
18
16
14
12
10
8
6
4
2
0
0
4
7
11
14
18
25
Time after administration (day)

Fig. 4

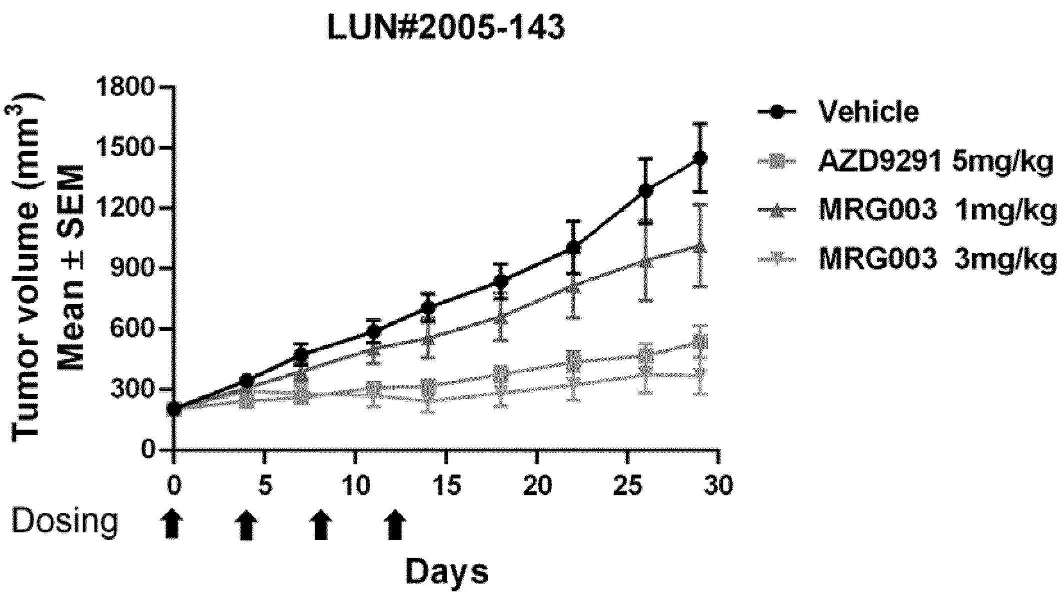
LUN#2005-143
Tumor volume (mm3)
Mean ± SEM
1800
1500
1200
900
600
300
0
0
5
10
15
20
25
30
Dosing
Days
Vehicle
AZD9291 5mg/kg
MRG003 1mg/kg
MRG003 3mg/kg

Fig. 5

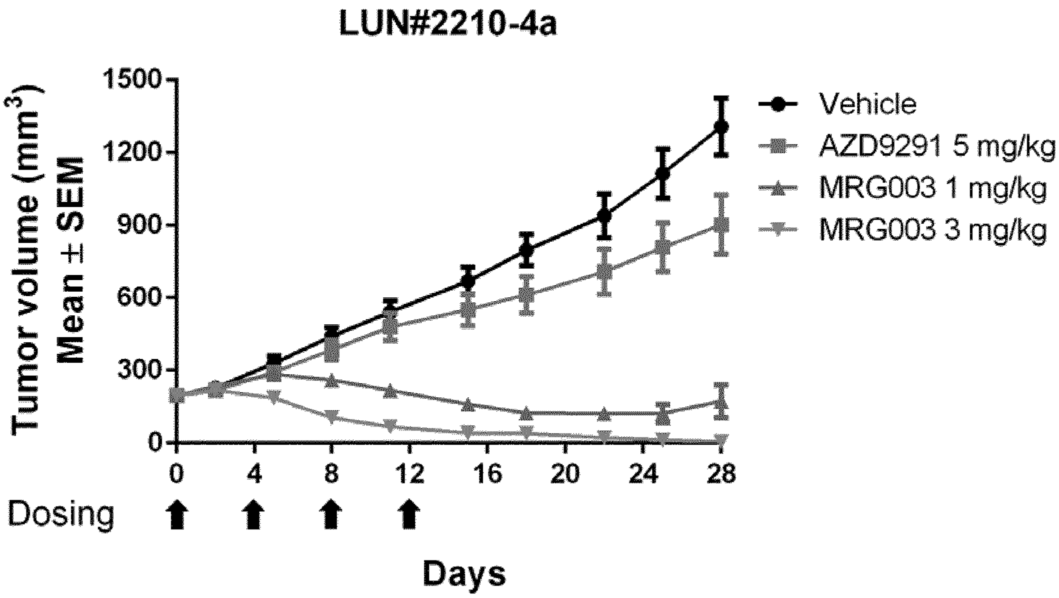
LUN#2210-4a
Tumor volume (mm3)
Mean ± SEM
1500
1200
900
600
300
0
0
4
8
12
16
20
24
28
Dosing
Days
Vehicle
AZD9291 5 mg/kg
MRG003 1 mg/kg
MRG003 3 mg/kg

Fig. 6

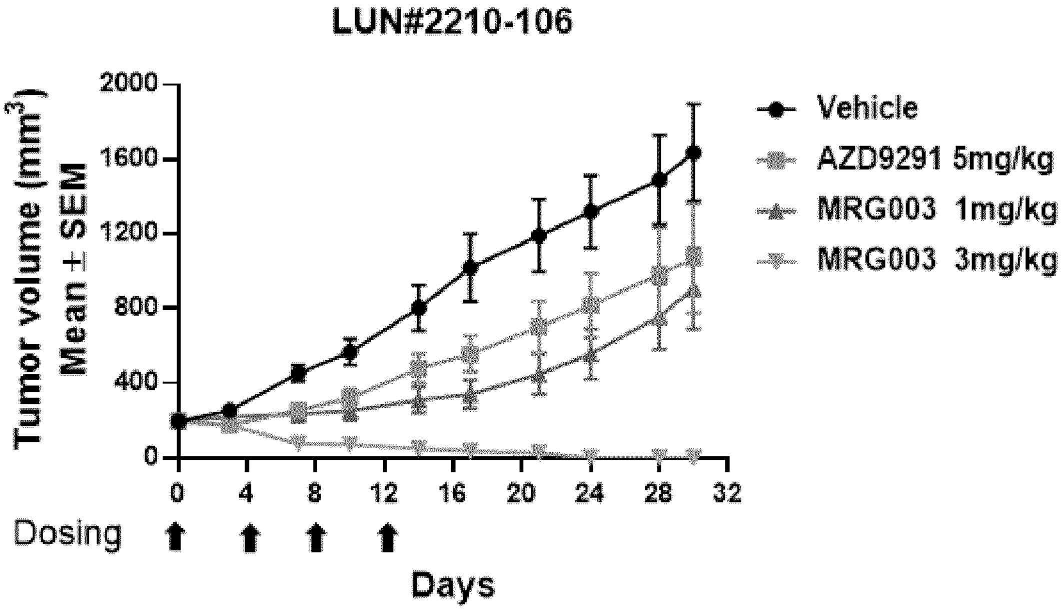
LUN#2210-106
Tumor volume (mm3)
Mean ± SEM
2000
1600
1200
800
400
0
0
4
8
12
16
20
24
28
32
Dosing
Days
Vehicle
AZD9291 5mg/kg
MRG003 1mg/kg
MRG003 3mg/kg

Fig. 7

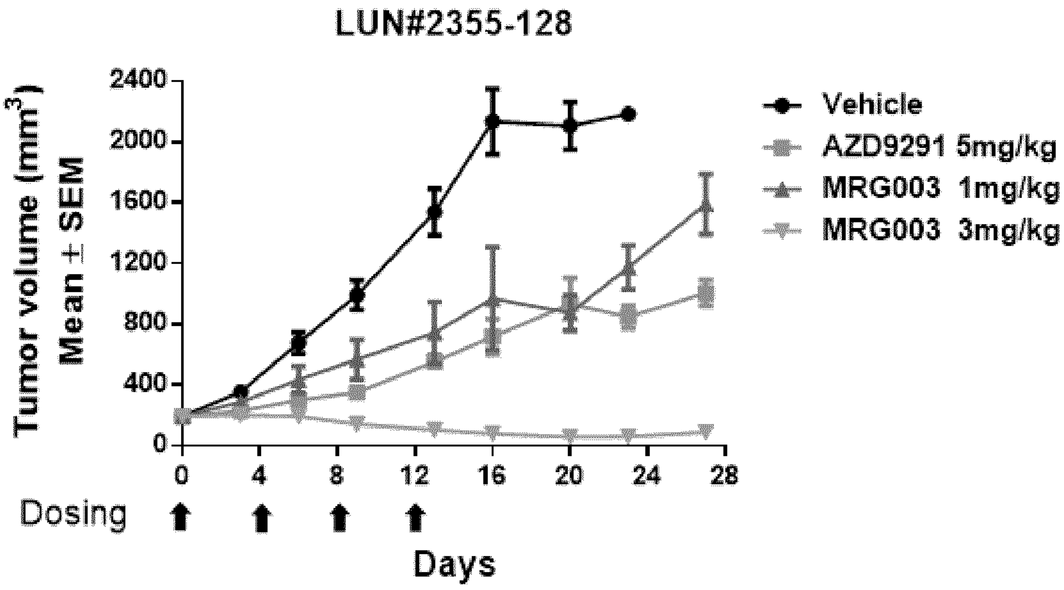
LUN#2355-128
Tumor volume (mm3)
Mean ± SEM
2400
2000
1600
1200
800
400
0
0
4
8
12
16
20
24
28
Dosing
Days
Vehicle
AZD9291 5mg/kg
MRG003 1mg/kg
MRG003 3mg/kg

Fig. 8

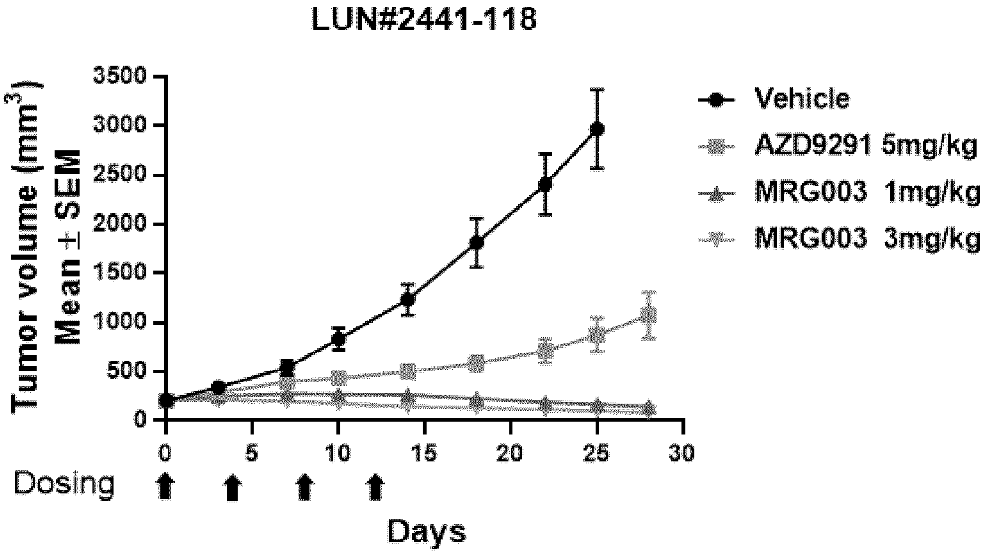
LUN#2441-118
Tumor volume (mm³)
Mean ± SEM
3500
3000
2500
2000
1500
1000
500
0
0
5
10
15
20
25
30
Dosing
Days
Vehicle
AZD9291 5mg/kg
MRG003 1mg/kg
MRG003 3mg/kg

Fig. 9

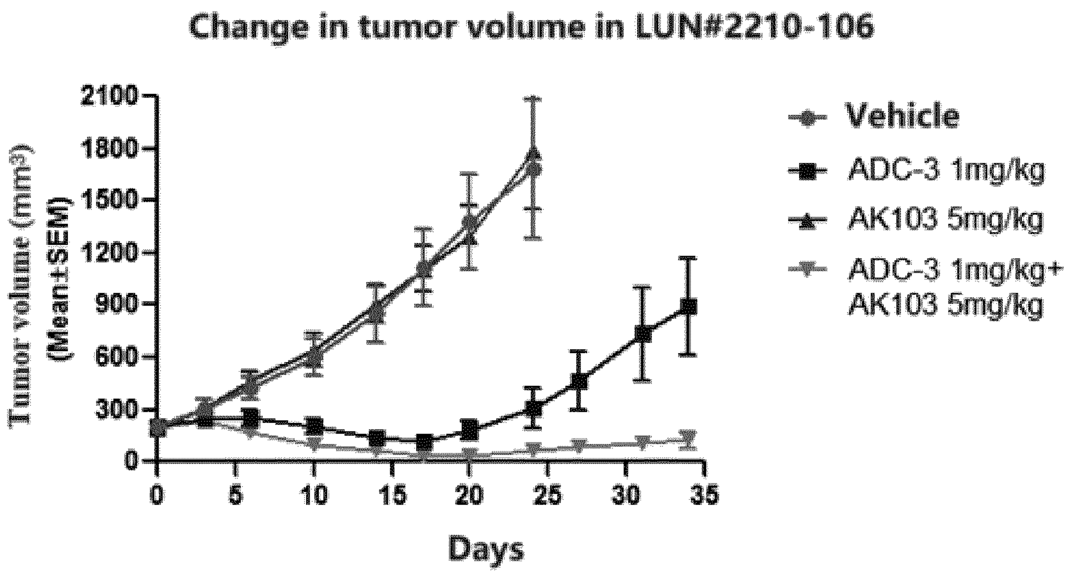
Change in tumor volume in LUN#2210-106
Tumor volume (mm³)
(Mean±SEM)
2100
1800
1500
1200
900
600
300
0
0
5
10
15
20
25
30
35
Days
Vehicle
ADC-3 1mg/kg
AK103 5mg/kg
ADC-3 1mg/kg+
AK103 5mg/kg

Fig. 10

**INTERNATIONAL SEARCH REPORT**

International application No.
**PCT/CN2022/118964**

**A. CLASSIFICATION OF SUBJECT MATTER**

A61K 47/68(2017.01)i; A61K 38/07(2006.01)i; A61K 39/395(2006.01)i; C07K 14/71(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; C07K; A61P

Documentation searched other than minimum documentation to the extent that such documents are included in the fields searched

Electronic data base consulted during the international search (name of data base and, where practicable, search terms used)

CNTXT; DWPI; ENTXTC; PUBMED; ELSEVIER; Patentics; NCBI; Web of Science; STN; 中国专利生物序列检索系统, China Patents Biological Sequence Search System: 上海美雅珂生物技术有限责任公司, 李虎, 胡朝红, 刘文超, 表皮生长因子, 偶联, 缀合, 非小细胞肺癌, 耐药, 突变, 马来酰亚氨基己酰基, 琥珀酰亚氨基, MCC, EGFR, SIAB, SPP, NSCLC, SHANGHAI MIRACOGEN, LI HU, HU CHAOHONG, LIU WENCHAO, SEQ ID NO: 5～18的序列, PD-1, PD-L1, Conjugate, non-small cell lung cancer, drug resistance, mutation

**C. DOCUMENTS CONSIDERED TO BE RELEVANT**

| Category* | Citation of document, with indication, where appropriate, of the relevant passages | Relevant to claim No. |
|---|---|---|
| A | CN 111529717 A (SHANGHAI MIRACOGEN INC.) 14 August 2020 (2020-08-14)<br>entire document | 1-10 |
| A | CN 102753580 A (ABBOTT BIOTHERAPEUTICS CORP. et al.) 24 October 2012 (2012-10-24)<br>entire document | 1-10 |
| A | CN 108864290 A (SHANGHAI JMT BIO INC.) 23 November 2018 (2018-11-23)<br>entire document | 1-10 |
| A | CN 103772504 A (SHANGHAI JMT BIO INC.) 07 May 2014 (2014-05-07)<br>entire document | 1-10 |

☐ Further documents are listed in the continuation of Box C.　☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 November 2022** | **24 November 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/118964**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

   [1] The actually submitted sequence table is an XML file of the standard ST.26.

Form PCT/ISA/210 (continuation of first sheet) (January 2015)

**INTERNATIONAL SEARCH REPORT**
**Information on patent family members**

International application No.
**PCT/CN2022/118964**

| Patent document cited in search report | Publication date (day/month/year) | Patent family member(s) | Publication date (day/month/year) |
|---|---|---|---|
| CN 111529717 A | 14 August 2020 | WO 2016131409 A1 | 25 August 2016 |
| | | US 2018036423 A1 | 08 February 2018 |
| | | CN 106999606 A | 01 August 2017 |
| CN 102753580 A | 24 October 2012 | TW 201122101 A | 01 July 2011 |
| | | ES 2639056 T3 | 25 October 2017 |
| | | WO 2011059762 A1 | 19 May 2011 |
| | | EP 2493929 A1 | 05 September 2012 |
| | | US 2011117110 A1 | 19 May 2011 |
| | | CA 2777825 A1 | 19 May 2011 |
| CN 108864290 A | 23 November 2018 | EP 3623388 A1 | 18 March 2020 |
| | | US 2020157223 A1 | 21 May 2020 |
| | | TW 201843181 A | 16 December 2018 |
| | | KR 20200003845 A | 10 January 2020 |
| | | AU 2018264321 A1 | 12 December 2019 |
| | | CN 113831417 A | 24 December 2021 |
| | | RU 2019138624 A | 09 June 2021 |
| | | WO 2018205936 A1 | 15 November 2018 |
| | | JP 2020520249 A | 09 July 2020 |
| CN 103772504 A | 07 May 2014 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103772504 A


### Non-patent literature cited in the description


- **KABAT.** Sequences of Proteins of Immunological Interest. National Institutes of Health, 1987
- **CHOTHIA ; LESK.** *J. Mol. Biol.,* 1987, vol. 196, 901-917
- **CHOTHIA et al.** *Nature,* 1989, vol. 342, 878-883
- **ALTSCHUL et al.** *Nucl. Acid. Res.,* 1977, vol. 25, 3389-3402
- **ALTSCHUL et al.** *J. Mol. Biol.,* 1990, vol. 215, 403-410
- **FOUCQUIER et al.** *Pharmacol. Res. Perspect.,* 2015, vol. 3 (3), e00149
- **WILD, R et al.** *Mol. Cancer Rher,* 2006, vol. 5 (1), 104-113
- Immunology-A Synthesis. Sinauer Associates, 1991
- Drug-To-Antibody (DAR) Ratio and Drug Distribution by Hydrophobic Interaction Chromatography and Reverse Phase High Performance Chromatography. **JUN OUYANG.** Antibody Drug Conjugates, Chapter 17, Methods in Molecular Biology. vol. 1045, 275-283